# EUROPEAN PATENT SPECIFICATION

(11) **EP 4 565 693 B1**
(45) Date of publication and mention of the grant of the patent: **18.11.2026**
(21) Application number: 23754739.3
(22) Date of filing: 04.08.2023
(51) Int. Cl.: C12N 15/10, C12Q 1/6806

(54) **METHODS OF PREPARING NORMALISED NUCLEIC ACID SAMPLES, KITS AND DEVICES FOR USE IN THE METHOD**
VERFAHREN ZUR HERSTELLUNG NORMALISIERTER NUKLEINSÄUREPROBEN SOWIE KITS UND VORRICHTUNGEN ZUR VERWENDUNG IN DIESEM VERFAHREN
MÉTHODES DE PRÉPARATION D'ÉCHANTILLONS D'ACIDES NUCLÉIQUES NORMALISÉS, TROUSSES ET DISPOSITIFS À UTILISER DANS CETTE MÉTHODE

(30) Priority: 04.08.2022 GB 202211385
(43) Date of publication of application: 11.06.2025
(73) Proprietor: WOBBLE GENOMICS LIMITED, Edinburgh, Lothian EH4 2HS (GB)
(72) Inventor: KUO, Richard Izen, Edinburgh EH4 2HS (GB)
(74) Representative: Boult Wade Tennant LLP
(86) International application number: PCT/EP2023/071740
(87) International publication number: WO 2024/028505

(56) References cited:
- WO-A1-2022/069039
- WO-A1-2022/069039
- US-A- 5 891 637
- US-A- 5 891 637
- US-B2- 7 049 065
- US-B2- 7 049 065
- MAHMOUDIAN REIHANEH ALSADAT ET AL: "Applying Subtractive Hybridization Technique to Enrich and Amplify Tumor-Specific Transcripts of Esophageal Squamous Cell Carcinoma", PATHOLOGY ONCOLOGY RESEARCH, TUD. KIADO, BUDAPEST, HU, vol. 23, no. 2, 15 July 2016 (2016-07-15), pages 271 - 279, XP036200892, ISSN: 1219-4956, [retrieved on 20160715], DOI: 10.1007/S12253-016-0090-5
- THOMAS MARK G. ET AL: "Sequencing of cDNA using anchored oligo dT primers", NUCLEIC ACIDS RESEARCH, vol. 21, no. 16, 1 January 1993 (1993-01-01), GB, pages 3915 - 3916, XP093101191, ISSN: 0305-1048, DOI: 10.1093/nar/21.16.3915
- JONASSON EMMA ET AL: "Total mRNA Quantification in Single Cells: Sarcoma Cell Heterogeneity", CELLS, vol. 9, no. 3, 19 March 2020 (2020-03-19), pages 759, XP093101199, DOI: 10.3390/cells9030759
- MORGANE BOONE ET AL: "Capturing the 'ome': the expanding molecular toolbox for RNA and DNA library construction", NUCLEIC ACIDS RESEARCH, vol. 46, no. 6, 5 March 2018 (2018-03-05), GB, pages 2701 - 2721, XP055681576, ISSN: 0305-1048, DOI: 10.1093/nar/gky167
- MAHMOUDIAN REIHANEH ALSADAT ET AL: "Applying Subtractive Hybridization Technique to Enrich and Amplify Tumor-Specific Transcripts of Esophageal Squamous Cell Carcinoma", PATHOLOGY ONCOLOGY RESEARCH, TUD. KIADO, BUDAPEST, HU, vol. 23, no. 2, 15 July 2016 (2016-07-15), pages 271 - 279, XP036200892, ISSN: 1219-4956, [retrieved on 20160715], DOI: 10.1007/S12253-016-0090-5
- THOMAS MARK G. ET AL: "Sequencing of cDNA using anchored oligo dT primers", NUCLEIC ACIDS RESEARCH, vol. 21, no. 16, 1 January 1993 (1993-01-01), GB, pages 3915 - 3916, XP093101191, ISSN: 0305-1048, DOI: 10.1093/nar/21.16.3915
- JONASSON EMMA ET AL: "Total mRNA Quantification in Single Cells: Sarcoma Cell Heterogeneity", CELLS, vol. 9, no. 3, 19 March 2020 (2020-03-19), pages 759, XP093101199, DOI: 10.3390/cells9030759
- MORGANE BOONE ET AL: "Capturing the 'ome': the expanding molecular toolbox for RNA and DNA library construction", NUCLEIC ACIDS RESEARCH, vol. 46, no. 6, 5 March 2018 (2018-03-05), GB, pages 2701 - 2721, XP055681576, ISSN: 0305-1048, DOI: 10.1093/nar/gky167

## Description

### FIELD OF THE INVENTION

The invention relates to methods and devices for preparing processed RNA samples. The invention also relates to detecting target nucleic acids. Methods of analysing biological samples using the processing and detection methods are also provided.

### BACKGROUND

RNA sequencing has become a powerful tool for understanding biology (Stark, R., Grzelak, M. & Hadfield, J. RNA sequencing: the teenage years. Nat. Rev. Genet. 20, 631-656 (2019)). Its applications range from drug development to improving agriculture. RNA sequencing is typically used for identifying differences between biological samples. These could be samples from infected and control animals to study disease resistance or samples from the same sample type over a time course to understand growth and development. The primary results generated from RNA sequencing are the discovery of all genes and isoforms that are expressed in a sample and the quantification of expression. Most cells and tissues share many of the same highly expressed genes which are commonly known as house-keeping genes. These genes are typically responsible for basic cell functions and thus do not provide cell specific characteristics. Since these house-keeping genes typically make up a large fraction of RNA within a sample, RNA sequencing data is usually dominated by sequencing reads from these non-informative RNA. This phenomenon results in two main negative effects on generating good results from RNA sequencing projects; first, genes and isoforms which are specific to the condition in question are difficult to detect, and second, the data generated is, in large part, redundant.

The first main negative effect has two consequences. The first is that the amount of sequencing required to detect genes of interest must be large enough to handle sampling inefficiencies caused by the low relative abundance of genes of interest. The second being that, in some cases, low abundance target genes may be simply impractical to identify. This can be evidenced by the still ongoing efforts to annotate the human genome where even after thousands of sequencing projects the full human transcriptome is still elusive with novel isoforms and genes being reported with regularity. Since eukaryotic transcriptomes derive their complexity from alternative splicing which generates combinatorial permutations, the search for novel RNA will likely be a constant endeavour.

These two consequences ultimately hamper scientific progress by limiting the abilities of researchers to produce ideal results from their sequencing experiments. These consequences also contribute to the impracticality of applying RNA sequencing toward a wider range of uses. For instance, for use in diagnostics and treatment tracking where the volume of sequencing required would be both time and cost prohibitive.

The second main negative effect (generation of redundant data) also has two main consequences. The first is that more data requires more processing time which increases overall cost and time of RNA sequencing experiments. These costs are both in terms of energy from additional computation required and work time from bioinformaticians that are tasked with processing the data. The second consequence is that redundant data results in the need for more storage. As sequencing is becoming more widespread, data storage has become a significant problem. For RNA sequencing technology to take on more roles, more efficient data generation is necessary to reduce storage requirements.

To address issues with high abundance house-keeping genes reducing sampling efficiency for genes of interest, complementary DNA (cDNA) normalization was developed (Alex S. Shcheglov, Pavel A. Zhulidov, Ekaterina A. Bogdanova, D. A. S. Normalization of cDNA Libraries, Nucleic Acids Hybrid. CHAPTER 5, (2014)). Since RNA sequencing typically relies on the conversion of RNA to double stranded cDNA, cDNA normalization takes advantage of the biochemical properties of cDNA to generate a uniform distribution of unique genes and isoforms within a cDNA library. In theory, the maximum non-targeted sampling efficiency is produced if all unique RNA sequences are represented at the same relative abundance. Thus the objective of normalization is to re-distribute a cDNA library to meet this criterion as closely as possible.

There are two forms of full length cDNA normalization that have been previously developed: the Duplex Specific Nuclease (DSN) method (Zhulidov, P. A. et al. Simple cDNA normalization using kamchatka crab duplex-specific nuclease. Nucleic Acids Res. 32, e37 (2004)) and the hydroxyapatite column method (Andrews-Pfannkoch, C., Fadrosh, D. W., Thorpe, J. & Williamson, S. J. Hydroxyapatite-mediated separation of double-stranded DNA, single-stranded DNA, and RNA genomes from natural viral assemblages. Appl. Environ. Microbiol. 76, 5039-5045 (2010)). Both methods rely on the denaturation and re-hybridization of cDNA strands. As the single stranded cDNA move about in solution, the sequences that are more highly abundant have a greater probability of finding a matching complementary sequence with which to re-hybridize. Thus, as re-hybridization reaches its limit, the remaining single stranded cDNA represents a normalized sequence library.

The difference between the two methods lies in their approach for isolating the single stranded cDNA library from the re-hybridized double stranded cDNA molecules.

In the DSN method, an enzyme which specifically cleaves double stranded DNA is used to decompose all double stranded cDNA within the solution. The solution is then purified and size-selected for cDNA sequences above a certain length. These sequences are then amplified using the Polymerase Chain Reaction (PCR).

In the column method, the denatured and re-hybridized cDNA library is passed through a heated column filled with hydroxyapatite granules. The hydroxyapatite preferentially binds to larger DNA molecules. The size of DNA that is bound is controlled by the concentration of phosphate buffer in which the cDNA library is dissolved. Thus the concentration of phosphate buffer must be tuned specifically for cDNA molecules within a certain range of sequence length. The cDNA is eluted through the column using increasing concentrations of phosphate buffer to extract increasing sizes of DNA molecules. Since the single stranded cDNA will be roughly one half the size of the re-hybridized cDNA, elution of the single stranded fraction can be managed if the mean cDNA sequence length is known. The resulting elution is intended to be enriched for the single stranded cDNA which are then amplified using PCR.

Since the DSN method uses enzymes which cleave all double stranded cDNA, in theory it can deplete low abundance sequences with segments that match high abundance sequences. This effect can also increase the probability of forming PCR chimeras. PCR chimeras are formed when incomplete single stranded cDNA sequences act as primers to other sequences thus combining the sequences in a way that does not occur in nature. PCR chimeras represent false positives for novel isoforms and are extremely challenging to distinguish from true alternative isoforms. Validating PCR chimeras typically requires in-depth biochemical assays. Both the depletion of low abundance sequences and the increased potential for PCR chimeras make the DSN method unsuitable for many RNA sequencing applications.

Since the column method only allows for segregation of high abundance and low abundance fractions within a narrow size range, it has significant bias against longer cDNA sequences. The effect of this is a loss of representation for longer RNA sequences. This effect makes it unsuitable for many RNA sequencing applications.

Accordingly, it is with these problems in mind that the present invention has been devised.

Mahmoudian et al. (2017) Pathol Oncol Res. 23(2):271-279 describes a subtractive hybridization technique to enrich and amplify tumor-specific transcripts of esophageal squamous cell carcinoma.

### SUMMARY OF THE INVENTION

RNA or cDNA samples are typically dominated by sequences from highly expressed genes which can negatively affect analysis of the samples. The present inventors have developed methods and devices for preparing processed nucleic acid samples with a more uniform distribution of sequences. A first nucleic acid sample is used to produce a probe set based on the intrinsic sequence abundances in the sample. Abundant sequences will produce more probes. When a second nucleic acid sample is applied to the probes more of the abundant sequences will bind to the probes enabling these sequences to be separated from the sample. In this manner the present invention enables normalization of full-length RNA. This technology has also been adapted for use in methods of extracting RNA and in the detection of specific target sequences. RNA processing according to the invention is also beneficial in methods of analyzing biological samples and diagnostic methods.

Aspects of the present invention are defined in the independent claims. Preferable features of the invention are defined in the dependent claims.

It should also be appreciated that options described in relation to one area of improvement will apply *mutatis mutandis* to other areas; e.g. sample types, nucleic acid types etc. as appropriate.

Described as reference, outside the scope of the claims, is a method for processing nucleic acid comprising:
(i) contacting a nucleic acid sample (e.g. an RNA sample) with an oligonucleotide array (e.g. a DNA, optionally a cDNA array), wherein one or more nucleic acid molecules from the nucleic acid sample anneal to one or more oligonucleotides of the oligonucleotide array; and
(ii) extracting the unannealed nucleic acid molecules thereby generating processed nucleic acid.

The array may be produced by a method comprising:
(i) bringing an RNA sample into contact with a surface, wherein the surface comprises two or more oligo-dT molecules, and wherein two or more RNA molecules from the RNA sample anneal to the oligo-dT molecules;
(ii) extending two or more of the oligo-dT molecules by reverse transcription using the annealed RNA molecules as templates to generate a DNA array comprising two or more cDNA molecules;
(iii) disassociating the annealed RNA molecules from the cDNA molecules;
(iv) removing the RNA sample from the surface.

The array may therefore comprise two or more oligonucleotides with sequences comprising oligo-dT followed by a cDNA sequence.

In further examples described as reference, outside the scope of the claims, the array was produced by a method comprising:
(i) denaturing a cDNA sample comprising double stranded cDNA molecules to produce single stranded cDNA molecules;
(ii) bringing the cDNA sample into contact with a surface, wherein the surface comprises two or more oligo-dT molecules, and wherein two or more cDNA molecules from the cDNA sample anneal to the oligo-dT molecules;
(iii) extending two or more of the oligo-dT molecules using the annealed cDNA molecules as templates to generate a DNA array comprising two or more DNA molecules;
(iv) disassociating the annealed cDNA molecules from the DNA molecules;
(v) removing the cDNA sample from the surface.

In these examples the array therefore comprises two or more oligonucleotides with sequences comprising oligo-dT followed by a DNA sequence.

Described herein, outside the scope of the claims, is a method for processing nucleic acid comprising:
(i) contacting a first nucleic acid sample with an oligonucleotide array, wherein the oligonucleotide array comprises two or more oligonucleotides linked to a surface, and wherein two or more nucleic acid molecules from the first nucleic acid sample anneal to the oligonucleotides of the oligonucleotide array;
(ii) extending two or more of the oligonucleotides using the annealed nucleic acid molecules as templates to generate a DNA array comprising two or more DNA molecules;
(iii) disassociating the annealed nucleic acid molecules from the DNA molecules;
(iv) removing the first nucleic acid sample from the surface;
(v) contacting a second nucleic acid sample with the DNA array, wherein one or more nucleic acid molecules from the second nucleic acid sample anneal to the DNA molecules; and
extracting the unannealed nucleic acid molecules thereby generating processed nucleic acid.

In examples outside the scope of the claims, any suitable nucleic acid molecule may processed using the devices, kits and methods described herein. The nucleic acid may be double stranded or single stranded. When the nucleic acid molecules are double-stranded, the double stranded nucleic acid molecules are first denatured to produce single stranded nucleic acid molecules.

In examples outside the scope of the claims, the nucleic acid may be DNA. The DNA may be genomic DNA, mitochondrial DNA, cDNA etc. cDNA is preferred. The DNA may be purified from any suitable sample. Sample types include blood samples (in particular from plasma, and also serum), other bodily fluids such as saliva, urine or lymph fluid. Other sample types include solid tissues, including frozen tissue or formalin fixed, paraffin embedded (FFPE) material. The DNA molecule may be a double-stranded DNA (dsDNA) molecule. In alternative examples, the DNA molecule is a single-stranded DNA (ssDNA) molecule. In some examples, ssDNA has already been
denatured *in situ* in the original sample. For example, the ssDNA may be purified from FFPE material. In further examples, the nucleic acid sample may comprise both ssDNA and dsDNA molecules. For instance, in the case of DNA purified from FFPE material, the DNA may include both ssDNA and dsDNA. The DNA may be found in, or derived from cells in a sample. Alternatively the DNA may be circulating, or "cell-free", DNA (cfDNA). Such DNA can be obtained from a range of bodily fluids including blood samples (in particular from plasma, and also serum), other bodily fluids such as saliva, urine or lymph fluid.

The nucleic acid is RNA. RNA may be obtained from the same sample types as DNA, as discussed above. The RNA may be messenger RNA (mRNA), microRNA (miRNA), transfer RNA (tRNA), ribosomal RNA (rRNA), long non-coding RNA (IncRNA), small interfering RNA (siRNA), small nucleolar RNA (snoRNA), piwi-interacting RNA (piRNA), tRNA-derived small RNA (tsRNA), small rDNA-derived RNA (srRNA), viral RNA etc. mRNA is preferred.

Thus, the invention provides a method for processing RNA comprising:
(i) contacting a first RNA sample with an oligonucleotide array, wherein the oligonucleotide array comprises two or more oligonucleotides linked to a surface, and wherein two or more RNA molecules from the first RNA sample anneal to the oligonucleotides of the oligonucleotide array;
(ii) extending two or more of the oligonucleotides by reverse transcription using the annealed RNA molecules as templates to generate a DNA array comprising two or more cDNA molecules;
(iii) disassociating the annealed RNA molecules from the cDNA molecules;
(iv) removing the first RNA sample from the surface;
(v) contacting a second RNA sample with the DNA array, wherein one or more RNA molecules from the second RNA sample anneal to the cDNA molecules; and
(vi) extracting the unannealed RNA molecules thereby generating processed RNA;
   wherein the first RNA sample and the second RNA sample are derived from the same RNA sample.

Described as reference, outside the scope of the claims, is a method for processing cDNA comprising
(i) denaturing a first cDNA sample comprising double stranded cDNA molecules to produce single stranded cDNA molecules;
(ii) contacting the first cDNA sample with an oligonucleotide array, wherein the oligonucleotide array comprises two or more oligonucleotides linked to a surface, and wherein two or more cDNA molecules from the first cDNA sample anneal to the oligonucleotides of the oligonucleotide array;
(iii) extending two or more of the oligonucleotides using the annealed cDNA molecules as templates to generate a DNA array comprising two or more DNA molecules;
(iv) disassociating the annealed cDNA molecules from the DNA molecules of the DNA array;
(v) removing the first cDNA sample from the surface;
(vi) denaturing a second cDNA sample comprising double stranded cDNA molecules to produce single stranded cDNA molecules;
(vii) contacting the second cDNA sample with the DNA array, wherein one or more cDNA molecules from the second cDNA sample anneal to the DNA molecules;
(viii) extracting the unannealed cDNA molecules thereby generating processed cDNA.

By "array" is meant a collection or arrangement of oligonucleotide (DNA, optionally cDNA) molecules linked or attached to a (solid) surface. Multiple methods of linking oligonucleotides to a surface are available (for example amine-modified oligonucleotides covalently linked to an activated carboxylate group or succinimidyl ester, thiol-modified oligonucleotides covalently linked via an alkylating reagent such as an iodoacetamide or maleimide, Digoxigenin NHS Ester, cholesterol-TEG, biotin-modified oligonucleotides captured by immobilized streptavidin) and are well-known to the skilled person. The link may be covalent or non-covalent. The link may be direct or indirect. The DNA array may be a cDNA array.

In specific embodiments the method for processing RNA reduces the variability in the levels of the RNA (e.g. by at least 10%, at least 20%, at least 30%, at least 40%, at least 50%, at least 60%, at least 70%, at least 80%, or at least 90%). The method for processing RNA may achieve a more uniform distribution of RNA sequences. The difference in abundance between the most abundant RNA and the least abundant RNA may be reduced (e.g. by at least 10%, at least 20%, at least 30%, at least 40%, at least 50%, at least 60%, at least 70%, at least 80%, or at least 90%). In certain embodiments the method for processing RNA reduces the number of molecules (copy number) of the (1, 10, 100, 1000, or 10000) most abundant RNA molecule(s) by at least 10%, at least 20%, at least 30%, at least 40%, at least 50%, at least 60%, at least 70%, at least 80%, or at least 90%. In specific embodiments the number of molecules (copy number) of the most abundant RNA molecule in the (second) RNA sample is reduced by at least 50% in the processed RNA. In further embodiments the relative abundance of the (1, 10, 100, 1000, or 10000) least abundant RNA molecule(s) is increased by at least 10%, at least 20%, at least 30%, at least 40%, at least 50%, at least 60%, at least 70%, at least 80%, or at least 90%. Thus, the method for processing RNA may be a method for normalizing RNA.

In specific examples described as reference, outside the scope of the claims, the method for processing cDNA reduces the variability in the levels of the cDNA (e.g. by at least 10%, at least 20%, at least 30%, at least 40%, at least 50%, at least 60%, at least 70%, at least 80%, or at least 90%). The method for processing cDNA may achieve a more uniform distribution of cDNA sequences. The difference in abundance between the most abundant cDNA and the least abundant cDNA may be reduced (e.g. by at least 10%, at least 20%, at least 30%, at least 40%, at least 50%, at least 60%, at least 70%, at least 80%, or at least 90%). In certain examples the method for processing cDNA reduces the number of molecules (copy number) of the (1, 10, 100, 1000, or 10000) most abundant cDNA molecule(s) by at least 10%, at least 20%, at least 30%, at least 40%, at least 50%, at least 60%, at least 70%, at least 80%, or at least 90%. In specific examples the number of molecules (copy number) of the most abundant cDNA molecule in the (second) cDNA sample is reduced by at least 50% in the processed cDNA. In further examples the relative abundance of the (1, 10, 100, 1000, or 10000) least abundant cDNA molecule(s) is increased by at least 10%, at least 20%, at least 30%, at least 40%, at least 50%, at least 60%, at least 70%, at least 80%, or at least 90%. Thus, the method for processing cDNA may be a method for normalizing cDNA.

In specific examples the method for processing nucleic acid reduces the variability in the levels of the nucleic acid (e.g. by at least 10%, at least 20%, at least 30%, at least 40%, at least 50%, at least 60%, at least 70%, at least 80%, or at least 90%). The method for processing nucleic acid may achieve a more uniform distribution of nucleic acid sequences. The difference in abundance between the most abundant nucleic acid and the least abundant nucleic acid may be reduced (e.g. by at least 10%, at least 20%, at least 30%, at least 40%, at least 50%, at least 60%, at least 70%, at least 80%, or at least 90%). In certain examples the method for processing nucleic acid reduces the number of molecules (copy number) of the (1, 10, 100, 1000, or 10000) most abundant nucleic acid molecule(s) by at least 10%, at least 20%, at least 30%, at least 40%, at least 50%, at least 60%, at least 70%, at least 80%, or at least 90%. In specific examples the number of molecules (copy number) of the most abundant nucleic acid molecule in the (second) nucleic acid sample is reduced by at least 50% in the processed nucleic acid. In further examples the relative abundance of the (1, 10, 100, 1000, or 10000) least abundant nucleic acid molecule(s) is increased by at least 10%, at least 20%, at least 30%, at least 40%, at least 50%, at least 60%, at least 70%, at least 80%, or at least 90%. Thus, the method for processing nucleic acid may be a method for normalizing nucleic acid.

In theory, the maximum non-targeted sampling efficiency is produced if all unique nucleic acid sequences are represented at the same relative abundance. Thus the objective of normalization is to re-distribute a nucleic acid sample to meet this criterion as closely as possible.

In certain embodiments processed RNA is RNA that is more readily analysable. It may be more efficiently sequenced because the relative representation of less abundant sequences is increased. Thus, the processed RNA may be normalized RNA.

In specific embodiments processed RNA comprises RNA sequences having substantially the same levels. For example, wherein the levels of the sequences of the processed RNA vary by less than 50%, less than 40%, less than 30%, less than 20%, or less than 10%. The processed RNA may be a processed RNA sample in which at least a portion of the (1, 10, 100, 1000, or 10000) most abundant sequence(s) in the second RNA sample have been removed.

In specific examples described as reference, outside the scope of the claims, processed cDNA comprises cDNA sequences having substantially the same levels. For example, wherein the levels of the sequences of the processed cDNA vary by less than 50%, less than 40%, less than 30%, less than 20%, or less than 10%. The processed cDNA may be a processed cDNA sample in which at least a portion of the (1, 10, 100, 1000, or 10000) most abundant sequence(s) in the second cDNA sample have been removed.

In specific examples processed nucleic acid comprises nucleic acid sequences having substantially the same levels. For example, wherein the levels of the sequences of the processed nucleic acid vary by less than 50%, less than 40%, less than 30%, less than 20%, or less than 10%. The processed nucleic acid may be a processed nucleic acid sample in which at least a portion of the (1, 10, 100, 1000, or 10000) most abundant sequence(s) in the second nucleic acid sample have been removed.

Described herein, outside the scope of the claims, is a method for preparing normalized RNA comprising:
(i) providing a first RNA sample;
(ii) bringing the first RNA sample into contact with a surface, wherein the surface comprises one or more oligonucleotides, and wherein one or more RNA molecules from the first RNA sample anneal with the one or more oligonucleotides;
(iii) reverse transcribing one or more RNA molecules using one or more of the oligonucleotides as a primer to generate one or more cDNA molecules;
(iv) disassociating the one or more RNA molecules from the one or more cDNA molecules;
(v) removing the first RNA sample;
(vi) providing a second RNA sample;
(vii) bringing the second RNA sample into contact with the surface, wherein one or more RNA molecules from the second RNA sample anneal with the one or more cDNA molecules; and
(viii) extracting the unannealed RNA molecules for use as a normalized RNA sample.

Described as reference, outside the scope of the claims, is a method for preparing normalized cDNA comprising
(i) providing a first cDNA sample comprising double stranded cDNA molecules;
(ii) denaturing the first cDNA sample to produce single stranded cDNA molecules;
(iii) bringing the first cDNA sample into contact with a surface, wherein the surface comprises one or more oligonucleotides, and wherein one or more cDNA molecules from the first cDNA sample anneal with the one or more oligonucleotides;
(iv) synthesising one or more probe DNA molecules using the one or more oligonucleotides as a primer and the one or more cDNA molecules as a template;
(v) disassociating the one or more cDNA molecules from the one or more probe DNA molecules;
(vi) removing the first cDNA sample;
(vii) providing a second cDNA sample comprising double stranded cDNA molecules;
(viii) denaturing the second cDNA sample to produce single stranded cDNA molecules;
(ix) bringing the second cDNA sample into contact with the surface, wherein one or more cDNA molecules from the second cDNA sample anneal with the one or more probe DNA molecules;
(x) extracting the unannealed cDNA molecules for use as a normalized cDNA sample.

Normalizing a nucleic acid sample results in production of a normalized nucleic acid sample. By "normalized" is meant that the levels of RNA or cDNA sequences in the sample are more equal. To achieve this the relative representation or levels of less abundant sequences may be increased and/or the relative representation or levels of more abundant sequences may be decreased. In specific examples normalized RNA or cDNA comprises RNA or cDNA sequences having substantially the same levels. For example, wherein the levels of the sequences of the normalized RNA or DNA vary by less than 50%, less than 40%, less than 30%, less than 20%, or less than 10%. The normalized RNA or cDNA may be a normalized RNA or cDNA sample in which at least a portion of the 10, 100, 1000, or 10000 most abundant sequences in the second RNA or cDNA sample have been removed. The methods for processing nucleic acid described herein may be methods for equalizing nucleic acid samples.

The methods described herein can be employed with both RNA and DNA, whereby the methods which use DNA are outside the scope of the claims. However, the use of double stranded cDNA requires a denaturation step to produce single stranded DNA molecules. A strand selection may also be employed as part of the processing of double stranded cDNA. Oligo-dT molecules will only bind to the cDNA strand comprising the poly (A) sequence.

Thus, the method for processing cDNA may further comprise following the last step (step (viii)):
(a) contacting the unannealed cDNA molecules with a second oligonucleotide array wherein the second oligonucleotide array comprises two or more oligo-dT molecules linked to a surface, and wherein one or more of the cDNA molecules anneal with the oligo-dT molecules;
(b) removing the unannealed cDNA molecules from the surface; and
(c) disassociating the annealed cDNA molecules from the oligo-dT molecules for use as a processed cDNA sample.

According to all aspects of the invention, in specific embodiments the oligonucleotide(s) are DNA molecules. According to all aspects of the invention, in specific embodiments the oligonucleotide(s) comprise oligo-dT sequences (optionally 2 to 200, 5 to 200, 2 to 100, 5 to 50, 7 to 25 or 12 to 18 nucleotides long). Thus, in certain embodiments the oligonucleotide(s) are oligo-dT molecule(s). By oligo-dT molecule is meant a molecule comprising a stretch of deoxythymidine. The oligo-dT molecule may be of any length appropriate to bind to the poly(A) tail (a sequence of adenine nucleotides) of messenger RNA or the second strand of a double stranded cDNA molecule. In certain embodiments, the oligo-dT molecule(s) are 2 to 100, 5 to 50, 7 to 25 or 12 to 18 nucleotides long. In further embodiments, the oligo-dT molecule(s) are at least 2, at least 5, at least 7, at least 12, at least 18 or at least 25 nucleotides long.

The oligonucleotide(s) may be immobilized on the surface. The surface may be two-dimensional such as a glass slides or three-dimensional such as micro-beads or microspheres. According to all aspects of the invention, in specific embodiments the surface is one or more beads or spheres, optionally magnetic beads. The methods of the invention may also be carried out in a microfluidic flowcell.

According to all aspects of the invention, in specific embodiments the RNA (the first RNA sample and/or the second RNA sample) comprises full length RNA.

In some embodiments, according to all aspects of the invention, the surface comprises two or more oligonucleotides and the oligonucleotides are optimally spaced so that the DNA molecules they prime do not interact with each other. Thus, in certain embodiments the oligonucleotides are optimally spaced so that the DNA (cDNA) molecules of the DNA array do not interact with each other. The optimal spacing for a given sample type may be determined based on the length of the DNA (cDNA) molecule expected to be produced.

This is in turn determined by the (maximum) length of the RNA molecules in the first RNA sample or biological sample (or in examples outside the scope of the clams, cDNA molecules in the first cDNA sample or nucleic acid molecules in the (first) nucleic acid sample).

The spacing between the oligonucleotides may be at least 1, at least 1.1, at least 1.2, at least 1.3, at least 1.4, at least 1.5, at least 1.6, at least 1.7, at least 1.8, at least 1.9, at least 2, at least 2.5, at least 3, at least 4 or at least 5 times the (maximum) length of the RNA molecules in the first RNA sample or biological sample (or in examples outside the scope of the claims, cDNA molecules in the first cDNA sample or nucleic acid molecules in the (first) nucleic acid sample). The spacing between the oligonucleotides may be between 1 and 5, between 1.3 and 3.5, between 1.4 and 3, or between 1.5 and 2.5 times the (maximum) length of the RNA molecules in the first RNA sample or biological sample (or in examples outside the scope of the claims, cDNA molecules in the first cDNA sample or nucleic acid molecules in the (first) nucleic acid sample). The spacing between the oligonucleotides may be 2 times the (maximum) length of the RNA molecules in the first RNA sample. or biological sample (or in examples outside the scope of the claims, cDNA molecules in the first cDNA sample or nucleic acid molecules in the (first) nucleic acid sample). In specific embodiments the spacing between the oligonucleotides is at least 2 times the (maximum) length of the RNA molecules in the first RNA sample.

In further embodiments the oligonucleotides are optimally spaced if the density of oligonucleotides (of the oligonucleotide array) is between 0.01 oligonucleotides per 1 micrometer squared and 10000 oligonucleotides per 1 micrometer squared, preferably between 0.1 oligonucleotides per 1 micrometer squared and 1000 oligonucleotides per 1 micrometer squared, more preferably between 1 oligonucleotide per 1 micrometer squared and 100 oligonucleotides per micrometer squared.

The first RNA sample and the second RNA sample are derived from the same RNA sample. In examples outside the scope of the claims, the first cDNA sample and the second cDNA sample may be derived from the same (biological) sample. In examples outside the scope of the claims, the first nucleic acid sample and the second nucleic acid sample may be derived from the same (biological) sample. Thus, from a given sample, for example a blood sample (processed to extract RNA), a portion of the RNA may be removed to form the first RNA sample and a further portion removed to form the second RNA sample. In examples outside the scope of the claims, from a given sample, for example a blood sample (optionally processed to generate cDNA), a portion may be removed to form the first cDNA sample and a further portion removed to form the second cDNA sample.

In examples outside the scope of the claims, from a given sample, for example a blood sample (optionally processed to extract nucleic acid), a portion may be removed to form the first nucleic acid sample and a further portion removed to form the second nucleic acid sample. The first RNA sample (or in examples outside the scope of the claims, first cDNA sample or first nucleic acid sample) and the second RNA sample (or in examples outside the scope of the claims, second cDNA sample or second nucleic acid sample) may be derived from the same species, organism, tissue and/or cell type. The first RNA sample (or in examples outside the scope of the claims, first cDNA sample or first nucleic acid sample) and the second RNA sample (or in examples outside the scope of the claims, second cDNA sample or second nucleic acid sample) may be derived from blood.

According to all aspects of the invention, in specific embodiments the method further comprises sequencing the processed RNA. In examples outside the scope of the claims, the method further comprises sequencing the processed cDNA or nucleic acid. The method for processing nucleic acid, cDNA or RNA may be a method for preparing nucleic acid, cDNA or RNA for sequencing. Sequencing may be RNA or DNA sequencing. In certain embodiments RNA is reverse transcribed to cDNA prior to sequencing. Sequencing may detect and/or quantify the (target) nucleic acid molecules. Such methods comprise processing according to the invention followed by sequencing of the processed products, optionally using a next generation sequencing (NGS) platform. Examples of NGS platforms include Illumina sequencing (such as Hi-Seq and Mi-Seq), SMRT sequencing (Pacific Biosciences), Nanopore sequencing, SoLID sequencing, pyrosequencing (e.g. Roche 454) and Ion-Torrent (Thermo Fisher) which are well-known to the skilled person.

The method of processing of the invention is also concerned with RNA extraction. Thus, there is provided a method for use in the method of the invention comprising:
(a) contacting a biological sample with an oligonucleotide array, wherein the oligonucleotide array comprises two or more oligonucleotides linked to a surface, wherein one or more RNA molecules from the biological sample anneal to the oligonucleotides of the oligonucleotide array;
(b) removing the unannealed sample from the surface; and
(c) disassociating the annealed RNA molecule(s) from the oligonucleotides to obtain an RNA sample.

This method may take place prior to step (i) of the method of processing of the invention.

Described herein, outside the scope of the claims, is a method comprising:
(a) bringing a biological sample into contact with a surface, wherein the surface comprises one or more oligonucleotides, wherein one or more RNA molecules from the biological sample anneal with the one or more oligonucleotides;
(b) removing the unannealed sample; and
(c) disassociating the one or more RNA molecules from the one or more oligonucleotides to obtain an RNA sample.

A portion of the obtained RNA sample forms the first RNA sample and a further portion forms the second RNA sample. The RNA sample may be reverse transcribed to cDNA. In certain embodiments the oligonucleotide(s) comprise one or more oligo-dT molecules. In specific embodiments the oligonucleotide(s) are oligo-dT molecules. Oligo-dT molecules will anneal with mRNA molecules with a poly(A) tail. In further embodiments, the oligonucleotide(s) comprise random or unique sequences to capture a range of RNAs in addition to mRNA. Custom oligonucleotide(s) may be designed to capture specific target RNA molecules (with complementary sequences). RNA molecules may be polyadenylated following extraction if they do not comprise a poly(A) tail.

After the processed RNA (or in examples outside the scope of the claims, the processed cDNA) is extracted, the method may further comprise disassociating the annealed RNA molecules from the cDNA molecules (or in examples outside the scope of the claims, the annealed cDNA molecules from the DNA molecules). The disassociated molecules may be removed (optionally disposed of) leaving a surface comprising the cDNA molecules (or in examples outside the scope of the claims, the DNA molecules). A further RNA or cDNA sample may then be processed using the surface. In specific embodiments the method for processing RNA further comprises, following step (vi) disassociating the annealed RNA molecules from the cDNA molecules and removing the disassociated RNA molecules from the surface and, optionally, repeating steps (v) and (vi) with a further RNA sample. In examples outside the scope of the claims, the method for processing cDNA further comprises, following step (viii) disassociating the annealed cDNA molecules from the DNA molecules and removing the disassociated cDNA molecules from the surface and, optionally, repeating steps (vi), (vii) and (viii) with a further cDNA sample.

According to all aspects of the invention, in specific embodiments the oligonucleotide(s) are at least 5 nucleotides, at least 10 nucleotides, at least 100 nucleotides, at least 200 nucleotides or at least 500 nucleotides in length. The oligonucleotide(s) may consist of 5 to 200 nucleotides. The oligonucleotide array or surface may comprise at least 10, at least 100, at least 1000, at least 10000, at least 100000 or at least 1 million oligonucleotides. The oligonucleotide array or surface may comprise at least 1.1, at least 1.2, at least, 1.3, at least 1.4, at least 1.5, at least, 1.6, at least 1.7, at least 1.8, at least 1.9, at least 2, at least 3, at least 4, at least 5, at least 10, at least 100, or at least 1000 times as many oligonucleotides as there are RNA molecules in the first and/or second RNA sample (or in examples outside the scope of the claims, cDNA molecules in the first and/or second cDNA sample or nucleic acid molecules in the nucleic acid sample). The oligonucleotide array or surface may comprise at least 10, at least 100, at least 1000, at least 10000, at least 100000 or at least 1 million oligonucleotides with unique sequences (i.e. no two sequences are identical). The oligonucleotide(s) may comprise sequences complementary to the 10, 20, 50, 100, 1000 or 10000 most abundant RNAs (mRNAs) in a given sample, optionally the 10, 20, 50, 100, 1000 or 10000 most abundant RNAs (mRNAs) in human blood. The oligonucleotide(s) may comprise one or more sequences complementary to the mRNA coding for human serum albumin, one or more alpha globulins (for example haptoglobin), one or more beta globulins (for example plasminogen) and/or one or more gamma globulins.

According to all aspects of the invention, in certain embodiments the amount of RNA molecules in the (first and/or second) RNA sample does not exceed the number of oligonucleotides in the oligonucleotide array (or in examples outside the scope of the claims, DNA molecules in the DNA array). In specific embodiments the amount of RNA molecules in the second RNA sample does not exceed the number of cDNA molecules in the DNA array.

Biological sample and sample are used interchangeably herein. According to all aspects of the invention, in specific embodiments the (biological) sample comprises a biological fluid or a fluid or lysate generated from a biological material. The biological fluid may comprise blood. In specific embodiments blood is processed on the same day as collection, no more than 72 hours after collection, no more than 2 weeks after collection, no more than 4 weeks after collection or 4-12 months after collection. In certain embodiments blood is stored at - 80°C prior to processing. Plasma, and also serum, samples are envisaged. In specific embodiments the sample is a human sample. Sample types include other biological fluids such as saliva, urine or lymph fluid. Other sample types include solid tissues, including frozen tissue or formalin fixed, paraffin embedded (FFPE) material. These samples may be processed to lyse cells.

The RNA may be messenger RNA (mRNA), transfer RNA (tRNA), ribosomal RNA (rRNA), long non-coding RNA (IncRNA), small interfering RNA (siRNA), small nucleolar RNA (snoRNA), piwi-interacting RNA (piRNA), tRNA-derived small RNA (tsRNA), small rDNA-derived RNA (srRNA), microRNA (miRNA), or viral RNA etc.

The invention also relates to the use of a device for performing the method of the invention.

Thus, the present invention relates to the use of an RNA processing device for producing processed RNA from a biological sample in a method for processing RNA according to the invention, the device comprising:
(i) a first module to receive the biological sample, wherein the first module comprises:
   (a) two or more oligonucleotides capable of annealing to one or more RNA molecules in the biological sample;
   (b) a first waste outlet to remove unannealed sample;
   (c) a sample outlet though which a portion of the sample comprising the one or more RNA molecules is capable of flowing following disassociation from the oligonucleotides; and
(ii) a second module to receive the one or more RNA molecules from the first module, wherein the second module comprises:
   (a) two or more oligonucleotides capable of annealing to one or more RNA molecules in the sample;
   (b) a processed RNA outlet through which the processed RNA can be obtained;
   (c) a waste RNA outlet to remove one or more RNA molecules;
wherein the first module and the second module together define a flow path along which a sample is capable of flowing.

The oligonucleotides may comprise oligo-dT sequences (optionally 2 to 200, 5 to 200, 2 to 100, 5 to 50, 7 to 25 or 12 to 18 nucleotides long). Thus, in certain embodiments the oligonucleotides of the first module and/or the oligonucleotides of the second module are oligo-dT molecules. By oligo-dT molecule is meant a molecule comprising a stretch of deoxythymidine. The oligo-dT molecule may be of any length appropriate to bind to the poly(A) tail (a sequence of adenine nucleotides) of messenger RNA or the second strand of a double stranded cDNA molecule. In certain embodiments, the oligo-dT molecule(s) are 2 to 100, 5 to 50, 7 to 25 or 12 to 18 nucleotides long.

In further embodiments, the oligonucleotides comprise random or unique sequences to capture a range of RNAs in addition to mRNA. Custom oligonucleotides may be designed to capture specific target RNA molecules (with complementary sequences).

In certain embodiments the oligonucleotides (oligo-dT molecules) of the first module are linked to a first surface and the oligonucleotides (oligo-dT molecules) of the second module are linked to a second surface. The first module may further comprise a sample inlet through which the biological sample is capable of entering the first module. In specific embodiments the first module further comprises a first reagent inlet through which reagents are capable of entering the first module and/or the second module further comprises a second reagent inlet through which reagents are capable of entering the second module. The RNA processing device may further comprise temperature control means for adjusting the temperature of the first module and/or the second module. In further embodiments, the first module comprises a flow cell and/or the second module comprises a flow cell. In specific embodiments the oligonucleotides are optimally spaced. Optimal spacing is discussed above. In specific embodiments the spacing between the oligonucleotides of the first module and/or the second module is at least 2 times the (maximum) length of the RNA molecules in the biological sample. In further embodiments the oligonucleotides are optimally spaced if the density of oligonucleotides (linked to the first and/or second surface) is between 0.01 oligonucleotides per 1 micrometer squared and 10000 oligonucleotides per 1 micrometer squared, preferably between 0.1 oligonucleotides per 1 micrometer squared and 1000 oligonucleotides per 1 micrometer squared, more preferably between 1 oligonucleotide per 1 micrometer squared and 100 oligonucleotides per 1 micrometer squared.

According to all aspects of the invention, in specific embodiments the RNA processing device further comprises a third module to receive the processed RNA, wherein the third module comprises reagents for preparing the processed RNA for sequencing. The RNA processing device may further comprise a fourth module to receive the RNA prepared for sequencing, wherein the fourth module comprises sequencing reagents.

A further aspect of the present invention provides use of an RNA processing device as described herein in a method of normalizing RNA.

In examples outside the scope of the claims, the method for processing nucleic acid may be a method for removing nucleic acid from a sample. The method for processing RNA may be a method for removing (abundant) RNA from the second RNA sample. In examples outside the scope of the claims, the method for processing cDNA may be a method for removing (abundant) cDNA from the second cDNA sample.

Where the surface or oligonucleotide array comprises one or more oligonucleotides with a sequence that is complementary to a target nucleic acid of interest, the target nucleic acid can bind to the one or more oligonucleotides. In this manner the target nucleic acid may be removed from a sample. The target nucleic acid may also be subjected to further processing such as sequencing.

Described herein, outside the scope of the claims, is a method for processing nucleic acid, the method comprising contacting a nucleic acid sample with a surface, wherein the surface comprises one or more oligonucleotides complementary to a target nucleic acid wherein the one or more oligonucleotides is at least 100 nucleotides in length and wherein the target nucleic acid anneals to the one or more oligonucleotides.

The oligonucleotide(s) may be at least 200 nucleotides in length, optionally at least 500 nucleotides in length. The surface may comprise two or more oligonucleotides. The oligonucleotide(s) may be linked to the surface.

According to all aspects of the invention, in specific embodiments the oligonucleotide(s) complementary to a target nucleic acid are complementary to the full length (or at least 70%, at least 80%, at least 90% of the full length) of the target nucleic acid.

Described herein, outside the scope of the claims, is an RNA processing device for producing processed RNA from a biological sample, the device comprising:
(i) a first module to receive the biological sample, wherein the first module comprises:
   (a) two or more oligo-dT molecules capable of annealing to one or more RNA molecules in the biological sample;
   (b) a first waste outlet to remove unannealed sample;
   (c) a sample outlet though which a portion of the sample comprising the one or more RNA molecules is capable of flowing following disassociation from the two or more oligo-dT molecules; and
(ii) a second module to receive the one or more RNA molecules from the first module, wherein the second module comprises:
   (a) one or more oligonucleotide molecules complementary to a target RNA in the sample;
   (b) an unannealed sample outlet to remove unannealed sample;
   (c) a target RNA outlet through which the target RNA can be obtained following disassociation from the one or more oligonucleotide molecules
wherein the first module and the second module together define a flow path along which a sample is capable of flowing.

In certain embodiments the one or more oligonucleotides complementary to the target RNA in the sample is at least 100 nucleotides in length, preferably at least 200 nucleotides in length, more preferably at least 500 nucleotides in length.

The target nucleic acid may be from an RNA virus. The target RNA may be (transcribed from) a bacterial gene such as an antibiotic resistance gene. The target RNA may be a biomarker for a disease.

The magnetic beads for use in the claimed methods can also be provided in the form of a kit. Thus in a related aspect the invention provides the use of a kit for processing an RNA sample in a method for processing RNA according to the invention, the kit comprising:
(a) one or more magnetic beads, wherein two or more oligo-dT molecules are linked to the one or more magnetic beads;
(b) a hybridization buffer; and
(c) a reverse transcriptase.

Any suitable reverse transcriptase may be included in the kit. Suitable buffers are also well known and commercially available.

A further aspect of the present invention provides use of a kit as described herein in a method of normalizing RNA.

Described as reference is a kit for processing a DNA sample, the kit comprising:
(a) one or more magnetic beads, wherein two or more oligo-dT molecules are linked to the one or more magnetic beads;
(b) a hybridization buffer; and
(c) a DNA polymerase.

Examples of DNA polymerases include thermostable polymerases such as Taq or Pfu polymerase and the various derivatives of those enzymes. Suitable buffers are also well known and commercially available.

Described as reference is the use of a kit as described herein in a method of normalizing cDNA.

Described herein, outside the scope of the claims, is a kit for detection of a target nucleic acid in a sample, the kit comprising:
(a) one or more magnetic beads comprising one or more oligonucleotides complementary to the target nucleic acid wherein the one or more oligonucleotides is at least 100 nucleotides in length; and
(b) a hybridization buffer.

The hybridization buffer may comprise HEPES 1M (pH = 7.5), NaCl 5M and H₂0.

The kits of the invention may further comprise one or more, up to all, of dinucleotide triphosphates (dNTPs), MgCl₂ and a buffer.

The oligonucleotide(s) may comprise sequences complementary to the 10, 20, 50, 100, 1000 or 10000 most abundant RNAs (mRNAs) in a given sample, optionally the 10, 20, 50, 100, 1000 or 10000 most abundant RNAs (mRNAs) in human blood. The oligonucleotide(s) may comprise one or more sequences complementary to the mRNA coding for human serum albumin, one or more alpha globulins (for example haptoglobin), one or more beta globulins (for example plasminogen) and/or one or more gamma globulins.

Methods of RNA extraction and processing may be combined and incorporated into pipelines for analysing biological samples.

Accordingly, the invention provides a method of analysing a biological sample from a subject, the method comprising:
(a) extracting RNA from the biological sample;
(b) preparing a processed RNA sample; and
(c) sequencing the processed RNA,
   wherein preparing a processed RNA sample comprises taking a portion of the extracted RNA to be a first RNA sample and a portion of the extracted RNA to be a second RNA sample and following the steps of the method for processing RNA of the invention.

The RNA may be full length RNA. The biological sample may comprise a biological fluid or a fluid or lysate generated from a biological material. In certain embodiments the biological sample is a liquid biopsy. In specific embodiments the biological sample is a blood sample, optionally a human blood sample.

In certain embodiments preparing a processed RNA sample comprises RNA normalization (reducing the variability in the levels of different RNA sequences in the sample). Thus, the processed RNA sample may be a normalized RNA sample. By "normalized" is meant that the levels of RNA sequences in the sample are more equal. To achieve this the relative representation or levels of less abundant sequences may be increased and/or the relative representation or levels of more abundant sequences may be decreased. In specific embodiments a normalized RNA sample comprises RNA sequences having substantially the same levels. For example, wherein the levels of the sequences of the normalized RNA sample vary by less than 50%, less than 40%, less than 30%, less than 20%, or less than 10%. The normalized RNA may be a normalized RNA sample in which at least a portion of the 10, 100, 1000, or 10000 most abundant sequences in the sample have been removed.

Preparing a processed RNA sample may comprise equalizing the RNA sample. Thus, in the processed RNA sample the relative abundance of all the unique RNA sequences may be more equal. For example, the levels of the unique sequences in the processed RNA sample may vary by less than 50%, less than 40%, less than 30%, less than 20%, or less than 10%.

In specific embodiments preparing a processed RNA sample reduces the variability in the levels of the RNA (e.g. by at least 10%, at least 20%, at least 30%, at least 40%, at least 50%, at least 60%, at least 70%, at least 80%, or at least 90%). Preparing a processed RNA sample may achieve a more uniform distribution of RNA sequences. In the processed RNA sample the difference in abundance between the most abundant RNA and the least abundant RNA may be reduced (e.g. by at least 10%, at least 20%, at least 30%, at least 40%, at least 50%, at least 60%, at least 70%, at least 80%, or at least 90%). In certain embodiments preparing a processed RNA sample reduces the number of molecules (copy number) of the (1, 10, 100, 1000, or 10000) most abundant RNA molecule(s) by at least 10%, at least 20%, at least 30%, at least 40%, at least 50%, at least 60%, at least 70%, at least 80%, or at least 90%. In specific embodiments the number of molecules (copy number) of the most abundant RNA molecule in the RNA sample is reduced by at least 50% in the processed RNA. In further embodiments the relative abundance of the (1, 10, 100, 1000, or 10000) least abundant RNA molecule(s) is increased by at least 10%, at least 20%, at least 30%, at least 40%, at least 50%, at least 60%, at least 70%, at least 80%, or at least 90% in the processed RNA.

In certain embodiments the processed RNA sample is more readily analysable. It may be more efficiently sequenced because the relative representation of less abundant sequences is increased.

In certain embodiments the method comprises diagnosing a disease in the subject.

Thus, the invention provides a method for diagnosing a disease in a subject, the method comprising:
(a) extracting RNA from a biological sample from the subject;
(b) preparing a processed RNA sample; and
(c) sequencing the processed RNA
   wherein preparing a processed RNA sample comprises taking a portion of the extracted RNA to be a first RNA sample and a portion of the extracted RNA to be a second RNA sample and following the steps of the method for processing RNA of the invention.

By diagnosing is meant determining that a subject has the disease at the time of testing.

In further embodiments the method comprises predicting a disease or identifying an increased risk of developing a disease. Described herein is a method for predicting a disease or identifying an increased risk of developing a disease in a subject, the method comprising:
(a) extracting RNA from a biological sample from the subject;
(b) preparing a processed RNA sample, according to the method of the invention; and
(c) sequencing the processed RNA.

By predicting is meant making a determination that a subject who at the time of testing does not have a disease is at an increased risk of developing a disease. The increased risk may be a risk higher than the average risk for the population. The increased risk may be a risk above a pre-calculated threshold level. The threshold level may be the point above which the benefits of increased monitoring and/or prophylactic treatment outweigh the negatives of potentially unnecessary intervention. The increased risk may be a percentage lifetime risk of greater than 1.5%, greater than 2%, greater than 5%, greater than 10%, greater than 50% or greater than 75%.

In yet further examples the method comprises selecting a treatment for a subject having a disease, predicting the responsiveness of a subject with a disease to a therapeutic agent and/or determining the clinical prognosis of a subject with a disease.

Sequencing the processed RNA allows the presence or absence and/or level of one or more RNA molecules to be determined. In certain embodiments the presence or absence of one or more RNA molecules in the processed RNA sample is used to identify whether the subject has the disease. In further embodiments the level of one or more RNA molecules in the processed RNA sample is used to identify whether the subject has the disease. A comparison with a reference point or value may be used to diagnose, or predict a clinical condition or outcome. While as few as one specific RNA molecule (an RNA molecule with a specific sequence) may be used to diagnose or predict a clinical prognosis or response to a therapeutic agent, the specificity and sensitivity or diagnosis or prediction accuracy may increase using more RNA molecules (of other specific sequences).

In certain embodiments the RNA extracted from the sample comprises cell-free RNA.

In certain embodiments extracting RNA from the biological sample comprises:
(a) contacting the biological sample with an oligonucleotide array wherein the oligonucleotide array comprises two or more oligonucleotides linked to a surface and wherein one or more RNA molecules from the sample anneals to the oligonucleotides of the oligonucleotide array;
(b) removing the unannealed sample from the surface; and
(c) disassociating the annealed RNA molecules from the oligonucleotides thereby generating an RNA sample.

The oligonucleotides may comprise one or more oligo-dT sequences. In specific embodiments the oligonucleotides are oligo-dT molecules.

Extracting RNA from the biological sample produces extracted RNA. In certain embodiments preparing a processed RNA sample comprises following the steps of the method(s) for processing RNA defined above. In further embodiments preparing a processed RNA sample comprises taking a portion of the extracted RNA formed by extracting RNA from the biological sample to be a first RNA sample and a portion of the extracted RNA to be a second RNA sample and following the steps of the method(s) for processing RNA defined above. Thus, in specific embodiments preparing a processed RNA sample comprises taking a portion of the extracted RNA to be a first RNA sample and a portion of the extracted RNA to be a second RNA sample and:
(i) contacting the first RNA sample with an oligonucleotide array, wherein the oligonucleotide array comprises two or more oligonucleotides linked to a surface, and wherein two or more RNA molecules from the first RNA sample anneal to the oligonucleotides of the oligonucleotide array;
(ii) extending two or more of the oligonucleotides by reverse transcription using the annealed RNA molecules as templates to generate a DNA array comprising two or more cDNA molecules;
(iii) disassociating the annealed RNA molecules from the cDNA molecules;
(iv) removing the first RNA sample from the surface;
(v) contacting the second RNA sample with the DNA array, wherein one or more RNA molecules from the second RNA sample anneal to the cDNA molecules; and
(vi) extracting the unannealed RNA molecules thereby generating processed RNA.

In further examples preparing a processed RNA sample comprises:
(i) contacting the extracted RNA with an oligonucleotide array (e.g. a DNA, optionally a cDNA array), wherein one or more RNA molecules from the extracted RNA anneal to one or more oligonucleotides of the oligonucleotide array; and
(ii) extracting the unannealed RNA molecules thereby generating processed RNA.

The method of analysing a biological sample from a subject may comprise use of one or more of the RNA processing device(s) and kit(s) of the present invention.

In specific embodiments sequencing the processed RNA comprises long-read sequencing.

Described herein is the use of a method, device or kit as described herein in a process of RNA or DNA sequencing, optionally for discovery of new RNA and/or detection of low abundance RNA, further optionally wherein the sequencing is single cell sequencing.

Described herein is the use of a method, device or kit as described herein in a process of metagenomic sequencing for discovery of new microbes and/or detection of low abundance microbes.

Described herein is the use of a method, device or kit as described herein in a process of screening RNA samples, or screening genetic samples for the presence of infectious diseases. Outside the scope of the claims, is the use of a method, device or kit as described herein in a process of screening DNA samples.

Described herein is the use of a method, device or kit as described herein in a process of detecting a nucleic acid biomarker, optionally a disease biomarker, further optionally a cancer biomarker.

In particular embodiments, according to all aspects of the invention, the method further comprises reporting the result. The result may be in the form of an RNA or DNA sequence, an indication of the presence or absence of a microbe or disease and/or an indication of the presence or absence or level of a disease biomarker.

### DESCRIPTION OF THE FIGURES

The above and other aspects of the present invention will now be described in further detail, by way of example only, with reference to the following examples and the accompanying figures.
**Figure 1** - A schematic representation of a magnetic bead with oligo-dT primers attached. During RNA processing according to the invention RNA molecules anneal to the oligo-dT molecules and reverse transcription creates a cDNA copy attached to the bead.
**Figure 2** - A schematic representation of a magnetic bead with cDNA probes attached. The magnetic bead is re-introduced to another batch of RNA. A portion of the RNA anneals to the probes (captured RNA). The beads are then immobilized and the solution extracted comprising the normalized RNA.
**Figure 3** - A schematic representation of a microfluidic flowcell in use for RNA processing according to the invention. RNA flows through and the temperature is cooled down to allow for poly-A annealing to the oligo-dT forest.
**Figure 4** - A schematic representation of a microfluidic flowcell in use for RNA processing according to the invention. Reverse transcription materials are added and incubation for reverse transcription carried out.
**Figure 5** - A schematic representation of a microfluidic flowcell in use for RNA processing according to the invention. Heating disassociates RNA which is then flushed out.
**Figure 6** - A schematic representation of a microfluidic flowcell in use for RNA processing according to the invention showing the cDNA forest ready for RNA to be normalized.
**Figure 7** - A schematic representation of a microfluidic flowcell in use for RNA processing according to the invention. New RNA is added and incubated between 45°C and 75°C (for example, at around 68°C) for association. The abundant RNA anneals to the cDNA forest while the free normalized RNA flows through.
**Figure 8** - A schematic representation of a microfluidic flowcell in use for RNA processing according to the invention. Heating to between 80°C and 100°C (for example, 98°C) disassociates RNA which flows through to waste. The cycle starting from Figure 7 can then be repeated.
**Figure 9** - A schematic representation of RNA extraction according to the invention. A sample with lysed cells flows over the surface. Cooling down the temperature allows for poly-A annealing to the oligo-dT forest. The flowcell is flushed leaving only bound RNA. Heating up disassociates RNA which flows through to the next step.
**Figure 10** - A schematic representation of an RNA processing device according to the invention.
**Figure 11** - A schematic representation of RNA processing according to the invention where the oligonucleotides linked to the surface are complementary to target RNA (designed probe cDNA forest). A sample with lysed cells flows through. Incubation between 45°C and 75°C (for example, at around 68°C) allows for full length association. The cell is flushed leaving only bound RNA. Heating up disassociates the RNA which flows though for further processing.
**Figure 12** - A schematic representation of DNA processing described herein as reference where the oligonucleotides linked to the surface are complementary to target DNA (designed probe cDNA forest). A sample with lysed cells and fragmented DNA flows through. Heating to between 80°C and 100°C (for example, 98°C) disassociates double strands. Incubation between 45°C and 75°C (for example, at around 68°C) allows for full length association. The cell is flushed leaving only bound DNA. Heating up disassociates the DNA which flows though for further processing.

### DETAILED DESCRIPTION

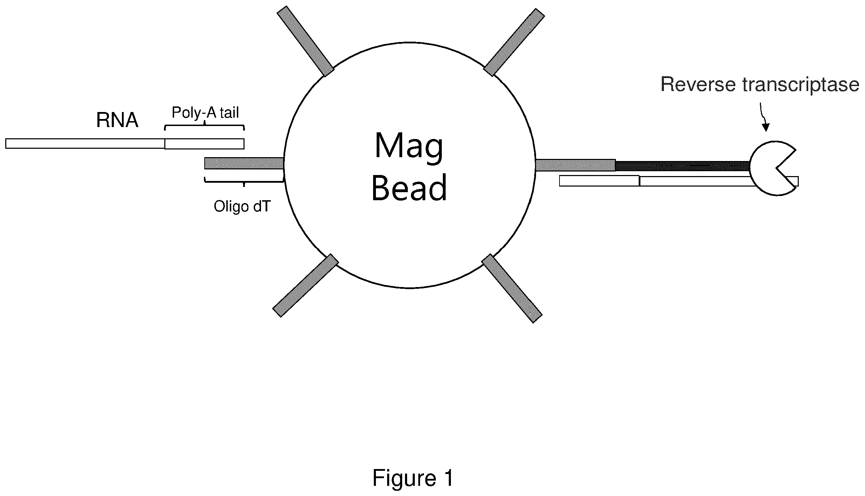

Figure 1 shows schematically an array of oligonucleotides, in this case oligo-dT molecules linked to a magnetic bead. A first RNA sample is contacted with the magnetic bead and RNA molecules comprising a poly-A tail anneal to the oligo-dT molecules. The oligo-dT molecules are extended by reverse transcription using the annealed RNA molecules as templates to generate cDNA molecules linked to the bead (a DNA array). Abundant RNA molecules (RNA sequences that occur more frequently in the sample) will produce more cDNA molecules. The annealed RNA molecules are disassociated from the cDNA molecules and the first RNA sample removed from the magnetic bead leaving the cDNA molecules linked to the bead.

This stage of the method to generate the cDNA molecules linked to the bead (DNA array) involves the following steps:
(i) Add magnetic beads with oligo-dT molecules to purified RNA solution (first RNA sample);
(ii) Heat up to between 65°C and 100°C (above 70°C, optionally between 70°C and 100°C, between 80°C and 100°C or between 90°C and 100°C) for 5 second to 1 minute to remove secondary structures;
(iii) Cool down to below 65°C (i.e. 65°C or below, for example between 30°C and 65°C) for 5 seconds to 1 minute to anneal oligo-dT molecules to poly-A tails of RNA;
(iv) Add reverse transcription materials (reverse transcriptase, dNTP, etc.);
(v) Incubate for reverse transcription (at between 30°C and 80°C, optionally at between 30°C and 60°C, for 1 minute to 2 hours);
(vi) Heat up to between 80°C and 100°C (for example, around 98°C) for 5 seconds to 1 minute to disassociate RNA from cDNA copy;
(vii) Immobilize beads using external magnet;
(viii) Remove liquid solution containing RNA.

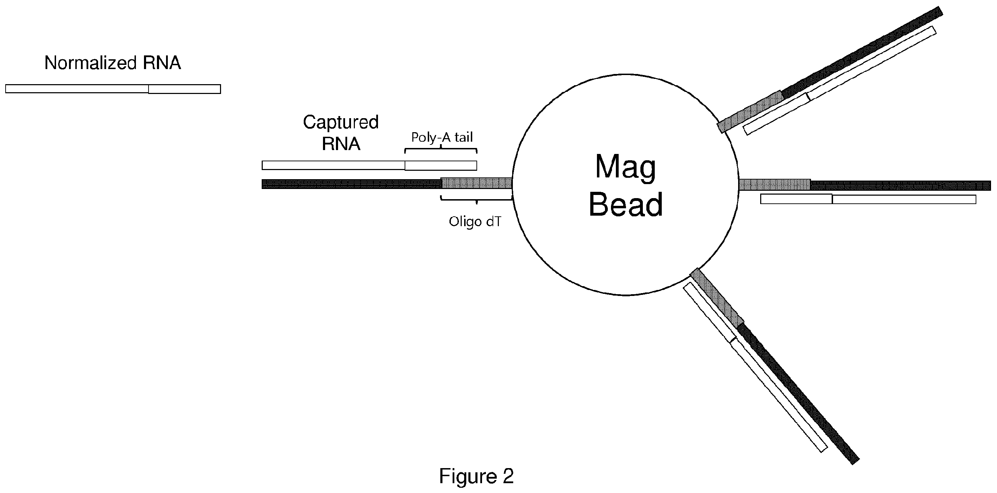

A second RNA sample is then contacted with the bead comprising the linked cDNA molecules. As shown in Figure 2, RNA molecules from the second RNA sample anneal to the cDNA molecules with the complementary sequence. As abundant RNA molecules produce more cDNA molecules in the stage shown in Figure 1, more of the abundant RNA molecules in the second RNA sample will be captured by the cDNA molecules then will be the case for the less abundant RNA molecules. The RNA molecules that do not anneal to the cDNA molecules, therefore, have a more uniform distribution of sequences - the RNA is normalized as it is no longer dominated by a few very abundant sequences. The magnetic bead is then immobilized and the unannealed RNA molecules are extracted thereby generating processed RNA.

The amount of RNA molecules in the second RNA sample should ideally not exceed the number of DNA molecules in the DNA array (cDNA forest) for each reaction cycle. If the DNA array outnumbers each pass of RNA it ensures there are enough probes to anneal to the high abundance RNA.

This stage of the method to generate the processed RNA involves the following steps:
(i) Add new aliquot of RNA solution (second RNA sample), optionally from the same sample;
(ii) Heat up to between 65°C and 100°C (above 70°C, optionally between 70°C and 100°C, between 80°C and 100°C or between 90°C and 100°C) for 5 seconds to 1 minute to remove secondary structures;
(iii) Cool down and incubate between 45°C and 75°C (for example, at around 68°C) for 10 second to 8 hours to allow for controlled full length association with cDNA;
(iv) Immobilize magnetic bead with external magnet;
(v) Extract liquid solution containing processed (normalized) RNA fraction;
(vi) Repeat with further aliquots of RNA solution to get more processed RNA (following disassociation of the annealed RNA).

The present invention makes possible the normalization of full length RNA. The advantages of analysing RNA directly include the fact that it is not necessary to do PCR (saves time and reagents and no PCR artefacts), lack of bias, nanopore sequencing can directly detect modifications present in RNA (modifications change the way in which RNA moves through pores).

The oligonucleotide array may be linked to any appropriate surface and the present invention is not limited to the use of magnetic beads. For example, the method may also be carried out in a microfluidic flowcell.

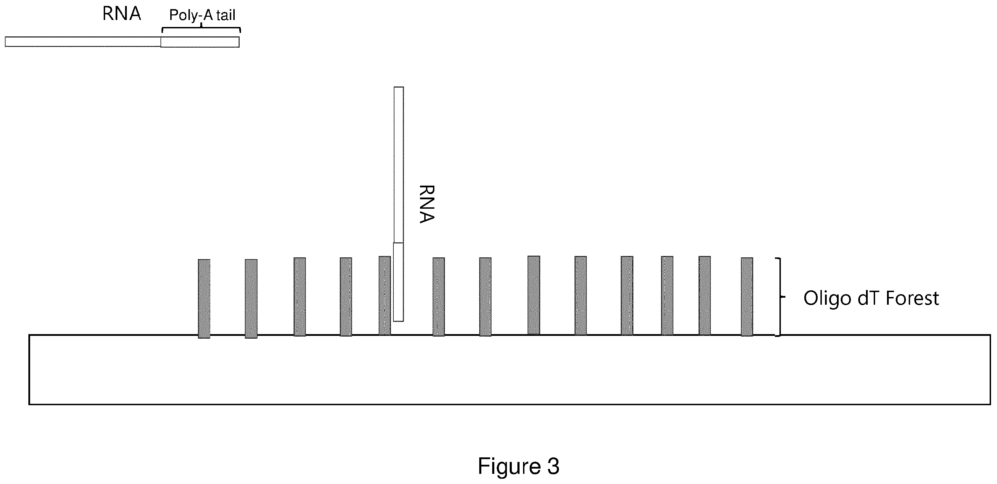

Figure 3 shows schematically an array of oligonucleotides, in this case oligo-dT molecules (also termed oligo-dT forest herein), linked to the surface of a flowcell. A first RNA sample flows through the flowcell and RNA molecules comprising a poly-A tail anneal to the oligo-dT molecules. The temperature is cooled down to below 65°C (i.e. 65°C or below, optionally between 30°C and 65°C) for the oligo-dT molecules to anneal to the poly-A tails of the RNA.

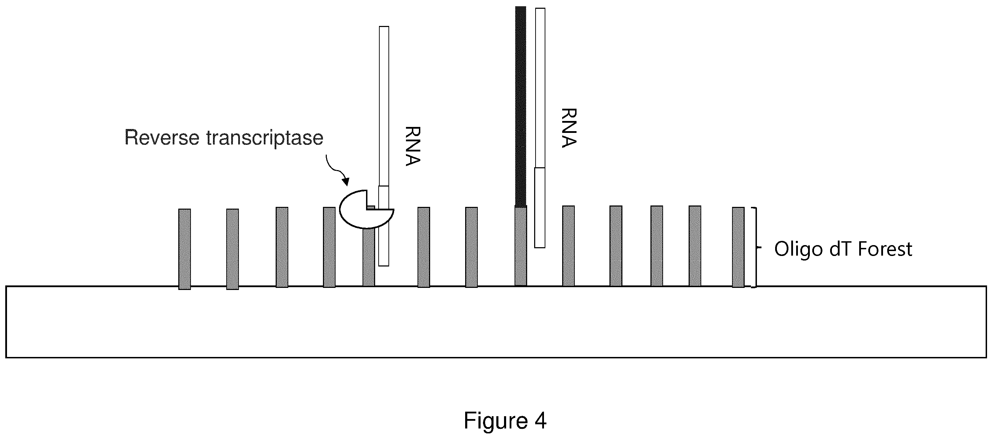

Reverse transcription reagents are added and incubation carried out. As shown in Figure 4 the oligo-dT molecules are extended by reverse transcription using the annealed RNA molecules as templates to generate cDNA molecules linked to the surface via the oligo-dT sequences (a DNA array). Abundant RNA molecules (RNA sequences that occur more frequently in the sample) will produce more cDNA molecules.

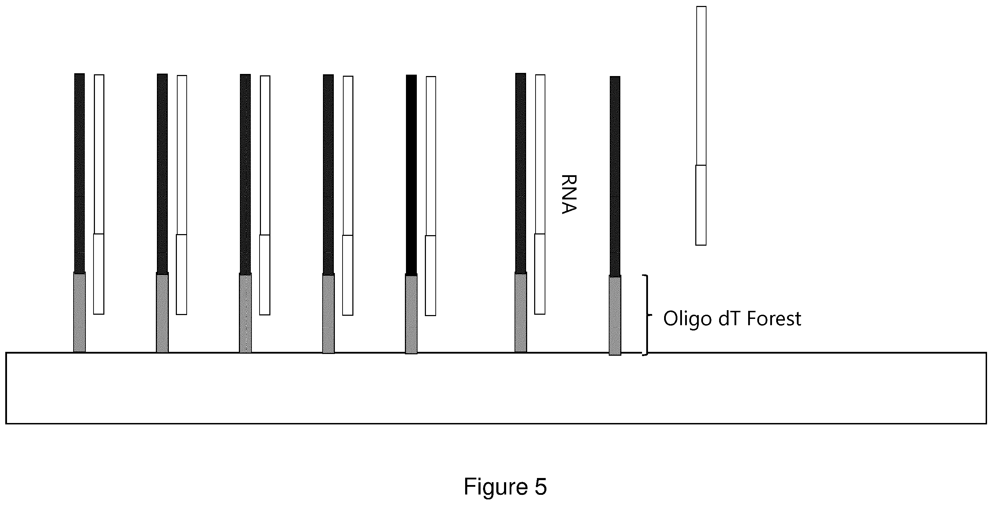

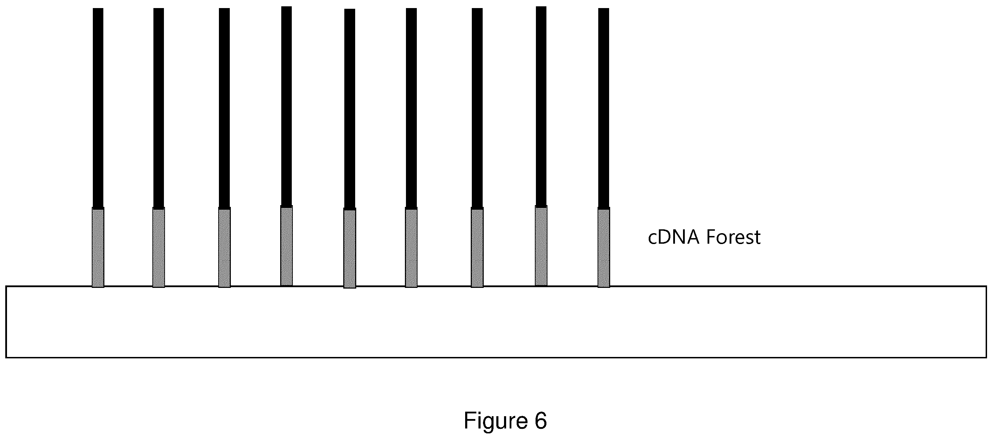

As shown in Figure 5 the annealed RNA molecules are disassociated from the cDNA molecules by heating. The first RNA sample is then flushed out leaving the cDNA molecules linked to the surface (DNA array or cDNA forest) as shown in Figure 6.

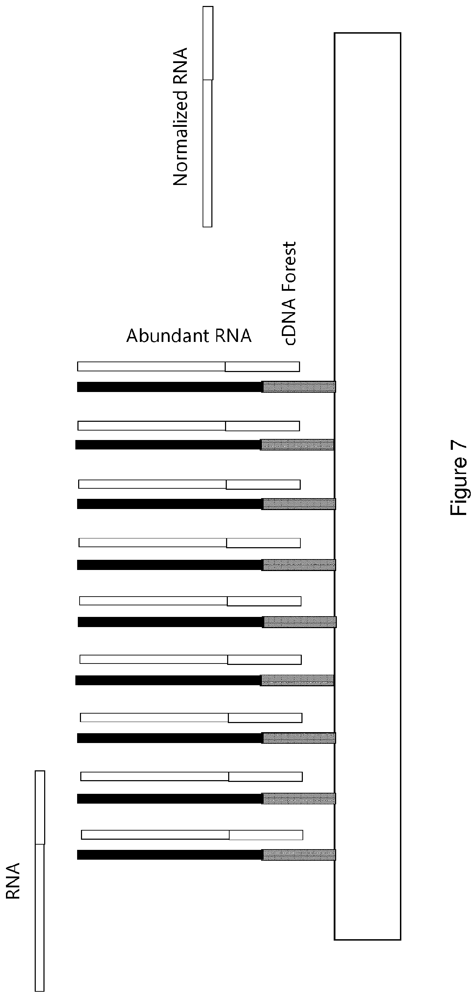

A second RNA sample is then contacted with the surface comprising the cDNA molecules and incubated at around 68°C. As shown in Figure 7, RNA molecules from the second RNA sample anneal to the cDNA molecules with the complementary sequence. As abundant RNA molecules produce more cDNA molecules in the step shown in Figure 4, more of the abundant RNA molecules in the second RNA sample will be captured by the cDNA molecules then will be the case for the less abundant RNA molecules. The RNA molecules that do not anneal to the cDNA molecules, therefore, have a more uniform distribution of sequences - the RNA is normalized as it is no longer dominated by a few very abundant sequences. The unannealed RNA molecules flow through thereby generating processed RNA.

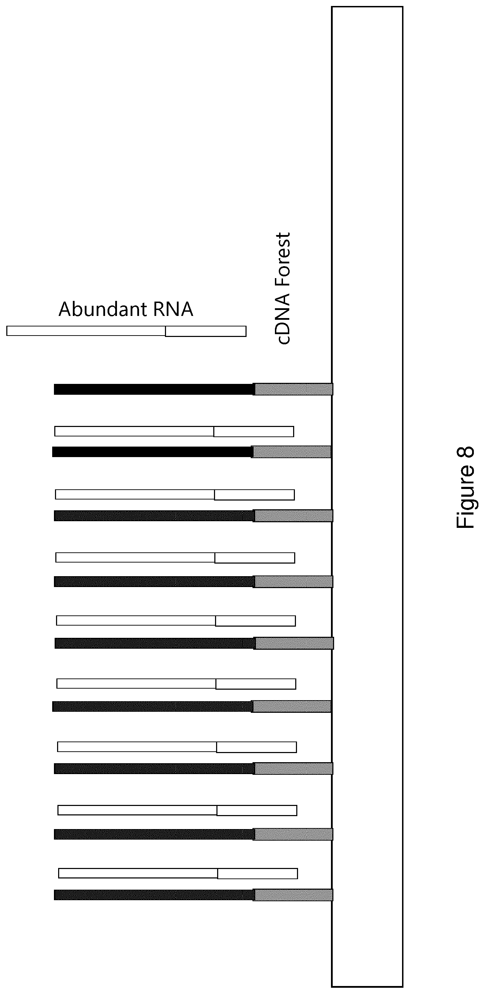

Figure 8 illustrates a further step of disassociating the annealed RNA molecules from the cDNA molecules by heating to 98°C. The disassociated RNA flows through to waste. The surface comprising the cDNA molecules can then be re-used with further RNA samples to generate more processed RNA.

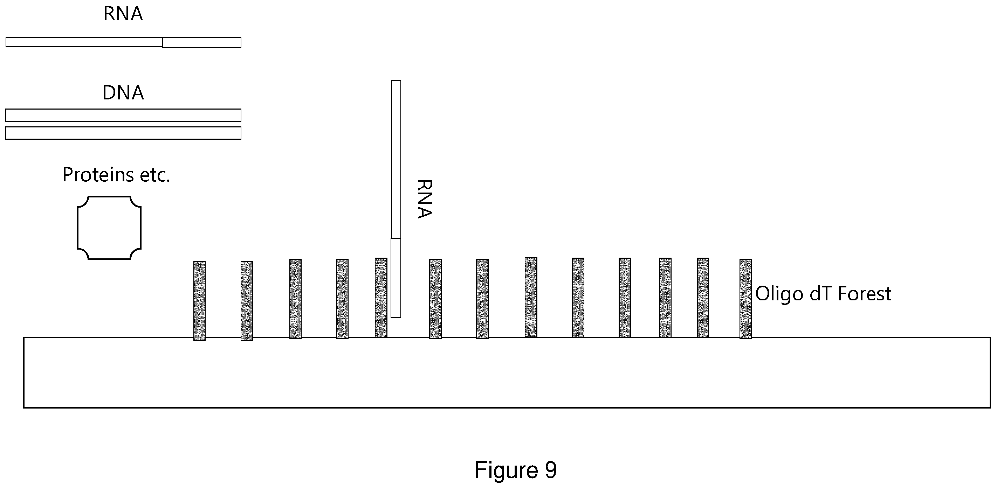

As illustrated in Figure 9 a similar principle can be applied to RNA extraction. A biological sample with lysed cells comprising RNA, DNA, proteins etc. flows over an array of oligonucleotides, in this case oligo-dT molecules (also termed oligo-dT forest herein) linked to the surface of a flowcell. The temperature is cooled down to below 65°C (i.e. 65°C or below, optionally between 30°C and 65°C) for the oligo-dT molecules to anneal to the poly-A tails of the RNA. The flowcell is flushed to leave only the annealed RNA. The temperature is then increased to between 80°C and 100°C (for example, 98°C) to disassociate the annealed RNA molecules from the oligonucleotides to obtain an RNA sample. The RNA then flows through for further processing.

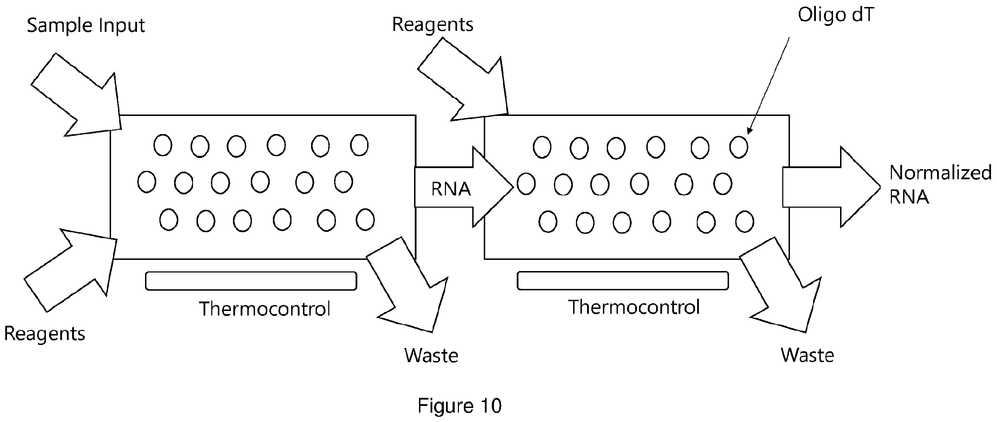

RNA extraction and RNA processing can be linked through combining microfluidic flowcells. One flowcell (also termed module or reaction chamber herein) extracts RNA which is then processed in a further flowcell (or module). An RNA processing device is illustrated schematically in Figure 10, which comprises two flowcells. The biological sample is input through a sample inlet in the first flowcell. The biological sample may be a sample of lysed cells comprising RNA, DNA, proteins etc. Reagents enter through a reagent inlet, for example buffer and/or RNA stabilising reagents. The surface of the first flowcell is as shown in Figure 9 i.e. an array of oligonucleotides, in this case oligo-dT molecules, linked to the surface of the flowcell. Both the first and second flowcells comprise temperature control means (thermocontrol) for adjusting the temperature. The temperature control means allow the temperature to be cooled down to below 65°C (i.e. 65°C or below, optionally between 30°C and 65°C) for the oligonucleotides to anneal to the RNA. The first flowcell comprises a first waste outlet to remove unannealed sample such that when the first flowcell is flushed only the annealed RNA is left. The temperature control means then allow the temperature to be increased to between 80°C and 100°C (for example, 98°C) to disassociate the annealed RNA molecules from the oligonucleotides to obtain an RNA sample. The first flow cell comprises a sample outlet though which RNA sample is capable of flowing following disassociation from the oligonucleotides. The first flowcell and the second flowcell together define a flow path along which the sample is capable of flowing. Thus, the first and second flowcells are joined by a connecter, for example a tube, that allows the RNA to flow from the first flowcell to the second flowcell for further processing.

The RNA sample enters through an RNA sample inlet in the second flowcell. The second flowcell (module) also comprises a second reagent inlet through which reagents are capable of entering the second flowcell. The surface of the second flowcell comprises an array of oligonucleotides, (for example oligo-dT molecules) linked to the surface of the flowcell. The RNA sample flows through the flowcell and RNA molecules anneal to the oligonucleotides. Reverse transcription reagents are added through the second reagent inlet. The oligonucleotides are extended by reverse transcription using the annealed RNA molecules as templates to generate cDNA molecules linked to the surface (a DNA array). The annealed RNA molecules are disassociated from the cDNA molecules by heating using the temperature control means. Thus, the temperature control means are capable of heating the RNA molecules to 98°C. The second flowcell comprises a waste RNA outlet to remove one or more RNA molecules. The waste RNA outlet allows the RNA sample to be flushed out leaving the cDNA molecules linked to the surface.

A further RNA sample then enters the second flowcell, contacts the surface comprising the linked cDNA molecules and is incubated between 45°C and 75°C (for example, at around 68°C). RNA molecules from the further RNA sample anneal to the cDNA molecules with the complementary sequence. The second flowcell comprises a processed RNA outlet through which the unannealed RNA molecules flow through thereby generating processed (normalized) RNA.

Where the surface or oligonucleotide array comprises one or more oligonucleotides with a sequence that is complementary to a target nucleic acid of interest, the target nucleic acid can bind to the one or more oligonucleotides. In this manner the target nucleic acid may be removed from a sample. The target nucleic acid may also be subjected to further processing such as sequencing. A further flowcell may be included in the RNA processing device discussed above that comprises one or more oligonucleotides with a sequence that is complementary to a target nucleic acid of interest. Alternatively the second flowcell may comprise one or more oligonucleotides with a sequence that is complementary to a target nucleic acid of interest.

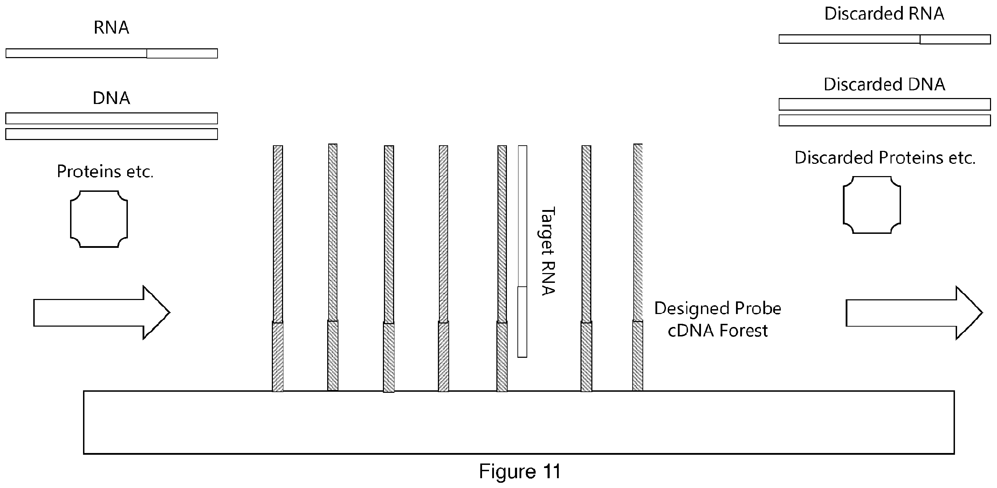

The target nucleic acid may also be directly extracted from a biological sample. Figure 11 shows a surface or array that comprises oligonucleotides complementary to a target RNA (also termed designed probe cDNA forest herein). The oligonucleotides are at least 100 nucleotides in length. A biological sample with lysed cells comprising RNA, DNA, proteins etc. flows over the array of oligonucleotides. Incubation between 45°C and 75°C (for example, at around 68°C) allows for full length association of target RNA with the oligonucleotides. The flowcell is flushed to leave only the annealed RNA. The temperature is then increased to between 80°C and 100°C (for example, 98°C) to disassociate the annealed RNA molecules from the oligonucleotides to obtain the target RNA. The RNA then flows through for further processing. The remaining RNA, DNA and protein may then be discarded or further processed.

The methods, devices and kits described herein (when used to process RNA) can be used to target RNA viruses, bacterial genes such as antibiotic resistance genes and RNA biomarkers for disease. Coupled with RNA sequencing this allows for precise diagnostics.

The DNA array (probe forest) can be reused. Thus the device can be used as a quick reusable screening for viral infection if coupled with (Nanopore) sequencing or another detection method (PCR, LAMP, etc.). The methods, kits and devices can also be used in agritech to monitor crops and livestock for diseases. Sample processing is fast and efficient.

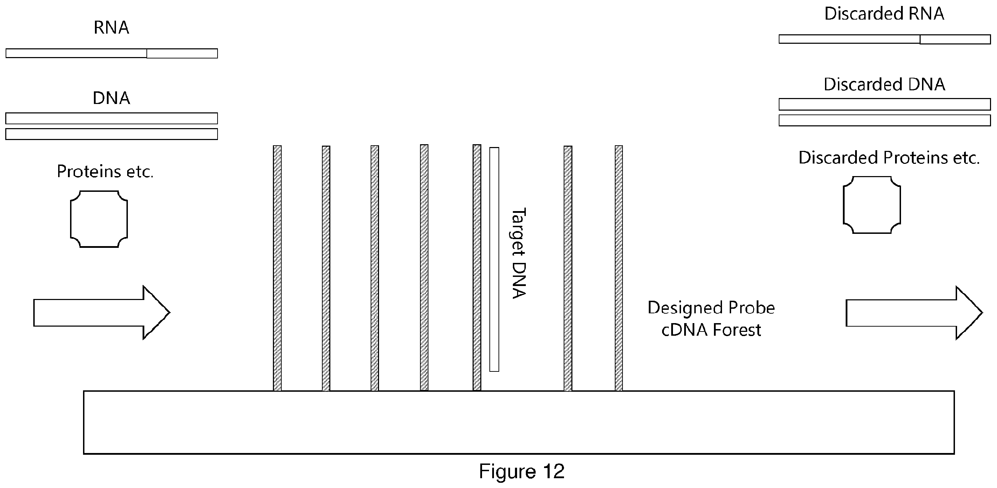

Outside the scope of the claims, the The methods, devices and kits described herein may also be used to process DNA. However, the use of double stranded DNA requires a denaturation step to produce single stranded DNA molecules. Figure 12 shows a surface or array that comprises oligonucleotides complementary to a target DNA (also termed designed probe cDNA forest herein). The oligonucleotides are at least 100 nucleotides in length. A biological sample with lysed cells comprising RNA, fragmented DNA, proteins etc. flows over the array of oligonucleotides. The sample is heated to between 80°C and 100°C (for example, 98°C) to disassociate the double stranded DNA. Then incubation between 30°C and 75°C (for example, at around 68°C) allows for full length association of target DNA with the oligonucleotides. The flowcell is flushed to leave only the annealed DNA. The temperature is then increased to between 80°C and 100°C (for example, 98°C) to disassociate the annealed DNA molecules from the oligonucleotides to obtain the target DNA. The target DNA then flows through for further processing. The remaining RNA, DNA and protein may then be discarded or further processed.

Outside the scope of the claims, the methods, devices and kits described herein (when used to process DNA) can be used to target DNA viruses, for bacterial identification (and identification of other microbes), to detect DNA biomarkers for disease and for rapid DNA identification as a means of validating individual identities. The DNA array (probe forest) can be reused. Thus the device can be used as a quick reusable screening for viral infection if coupled with (Nanopore) sequencing or another detection method (PCR, LAMP, etc.). The methods, kits and devices can also be used in agritech to monitor crops and livestock for diseases. Sample processing is fast and efficient.

Where oligonucleotide(s) complementary to a target nucleic acid are employed they may be complementary to the full length (or at least 70%, at least 80%, or at least 90% of the full length) of the target nucleic acid. This differs from typical probe based systems which only use a short oligonucleotide sequence to target nucleic acid.

Within the DNA array (cDNA forest) there is an optimum distance between the DNA molecules so that they do not interact with each other. This distance is influenced by the length of the cDNA expected so that it is optimal that any two points need to be about twice the length of the longest cDNA from each other. For example, when the biological sample is (human) blood, the maximum length of RNA is around 5 kb so the maximum length of the cDNA produced therefrom will be around 5 kb. Thus, at least 10 kb (6000 nm) would be the optimal spacing between the oligonucleotides in the oligonucleotide array and/or cDNA molecules in the DNA array. Where oligonucleotides complementary to a target DNA or RNA are used, the distance between the oligonucleotides can be smaller as the known sequences allow for designing of the oligonucleotide sequences so there is minimal interaction. Accordingly where oligonucleotides complementary to a target DNA or RNA are used, the distance between the oligonucleotides may be at least 1.1, at least 1.2, at least 1.3, at least 1.4, at least 1.5, at least 1.6, at least 1.7, at least 1.8 or at least 1.9 times the length of the oligonucleotides.

As noted above the density of oligonucleotides in the oligonucleotide array influences the density of DNA molecules in the DNA array. Thus, one means of preventing the DNA molecules in the DNA array from interacting with each other is using a certain spacing (i.e. a maximum density) of oligonucleotides in the array as discussed above. The density of DNA molecules in the DNA array is also influenced by the concentration of RNA or cDNA molecules in the first RNA or first cDNA sample respectively. This concentration influences how many oligonucleotides in the oligonucleotide array capture an RNA molecule or cDNA molecule, as appropriate. This in turn influences how many DNA molecules are synthesised using the captured RNA or DNA as a template. Thus, the concentration of RNA or cDNA molecules in the first RNA or first cDNA sample may be adjusted to prevent the DNA molecules in the DNA array from interacting with each other.

Performance is also based on the ratio between the RNA and the DNA array (cDNA forest). Thermal control and kinetic control are relevant to optimum performance. A micropump can be used to generate laminar flow or turbulent flow.

Methods of RNA extraction and processing as described above may be combined and incorporated into pipelines for analysing biological samples. The method comprises:
(a) extracting RNA from the biological sample;
(b) preparing a processed RNA sample; and
(c) sequencing the processed RNA.

Blood or another liquid biopsy sample is collected from the subject in a container with cell lysis buffer and RNA stabilizing reagents. RNA stabilizing reagents are commercially available and include RNAlater^{®} (Sigma-Aldrich) and RNAprotect (Qiagen). A suitable buffer contains EDTA, sodium citrate and ammonium sulfate. Incubation for cell lysis can be, for example, 1 minute to 3 hours. The sample is then added to an RNA processing device as described above.

First RNA extraction (purification) takes place. The first reaction chamber (also termed flowcell or module herein) purifies the solution for RNA using oligonucleotides that can either be oligo-dT or random sequences. These oligonucleotides are bound to the surface of the chamber to make an oligo-forest. After the sample solution is pumped into the first chamber the chamber is heated to somewhere between 30°C and 75°C (optionally between 30°C and 65°C or between 60°C and 65°C) to allow for annealing of RNA to the oligo-forest. After the incubation period the remaining fluid is flushed out to a waste channel. The chamber is then heated to above 75°C (for example, between 80°C and 100°C) to release the remaining annealed RNA. This is then pumped through to the second reaction chamber.

The next step is preparing a processed RNA sample, in this case RNA normalization. The second reaction chamber (flowcell, module) has another oligo-forest. The purified RNA is cooled down in this chamber to below 65°C (i.e. 65°C or below, for example between 30°C and 65°C), optionally below 60°C, to allow for annealing to the oligo-forest. Reverse transcriptase and buffer is then added to create a complementary DNA strand using the oligo-forest as primers. Once the reverse transcription is completed the chamber is heated to between 80°C and 100°C (optionally above 90°C) to disassociate the RNA from the cDNA-forest. The solution is then flushed to waste channel. Another sample of purified RNA is then pumped into the second chamber with the cDNA-forest. The chamber is heated to between 45°C and 75°C (optionally between 60°C and 75°C) to allow for full length annealing of RNA to cDNA.

After incubation the non-annealed RNA is pumped into the collection chamber for further processing (to be sequenced). The second chamber is then heated to between 80°C and 100°C (optionally above 90°C) to release the RNA. The disassociated RNA is flushed to the waste channel. This process is repeated until an adequate amount of normalized RNA is produced for sequencing.

The next stage is preparation for sequencing. The normalized RNA can then be pumped into additional reaction chambers (flowcells, modules) which will prepare the sequencing libraries. Depending on the sequencing technology this could involve the ligation of adapters, second strand synthesis and/or any other required modifications to allow for sequencing. The sequencing libraries will then be pumped into a sequencing chamber for sequencing.

The next stage is sequencing and data processing. During sequencing the raw data can be uploaded to cloud servers for data processing and archiving.

Combining RNA extraction and processing in this way provides a device that can be used for immediate processing of blood or other samples minimizing issues with RNA degradation.

## Claims

1. A method for processing RNA comprising:
(i) contacting a first RNA sample with an oligonucleotide array, wherein the oligonucleotide array comprises two or more oligonucleotides linked to a surface, and wherein two or more RNA molecules from the first RNA sample anneal to the oligonucleotides of the oligonucleotide array;
(ii) extending two or more of the oligonucleotides by reverse transcription using the annealed RNA molecules as templates to generate a DNA array comprising two or more cDNA molecules;
(iii) disassociating the annealed RNA molecules from the cDNA molecules;
(iv) removing the first RNA sample from the surface;
(v) contacting a second RNA sample with the DNA array, wherein one or more RNA molecules from the second RNA sample anneal to the cDNA molecules; and
(vi) extracting the unannealed RNA molecules thereby generating processed RNA;
wherein the first RNA sample and the second RNA sample are derived from the same RNA sample.

2. The method of claim 1 wherein:
(i) the oligonucleotides comprise oligo-dT sequences;
(ii) the surface is one or more magnetic beads;
(iii) the method is carried out in a microfluidic flowcell;
(iv) the first RNA sample and/or the second RNA sample comprises full length RNA; and/or
(v) the oligonucleotides are optimally spaced so that the cDNA molecules of the DNA array do not interact with each other.

3. The method of claim 1 or claim 2 further comprising sequencing the processed RNA.

4. The method of any of claims 1 to 3 further comprising, prior to step (i):
(a) contacting a biological sample with an oligonucleotide array, wherein the oligonucleotide array comprises two or more oligonucleotides linked to a surface, and wherein one or more RNA molecules from the biological sample anneal to the oligonucleotides of the oligonucleotide array;
(b) removing the unannealed sample from the surface; and
(c) disassociating the annealed RNA molecule(s) from the oligonucleotides to obtain an RNA sample.

5. The method of any of claims 1 to 4 further comprising, following step (vi) disassociating the annealed RNA molecules from the cDNA molecules and removing the disassociated RNA molecules from the surface and, optionally, repeating steps (v) and (vi) with a further RNA sample.

6. The method of claim 4 or claim 5 wherein the biological sample comprises a biological fluid or a fluid or lysate generated from a biological material.

7. Use of an RNA processing device for producing processed RNA from a biological sample in a method according to any of claims 1 to 6, the device comprising:
(i) a first module to receive the biological sample, wherein the first module comprises:
(a) two or more oligonucleotides capable of annealing to one or more RNA molecules in the biological sample;
(b) a first waste outlet to remove unannealed sample;
(c) a sample outlet though which a portion of the sample comprising the one or more RNA molecules is capable of flowing following disassociation from the oligonucleotides; and
(ii) a second module to receive the one or more RNA molecules from the first module, wherein the second module comprises:
(a) two or more oligonucleotides capable of annealing to one or more RNA molecules in the sample;
(b) a processed RNA outlet through which the processed RNA can be obtained;
(c) a waste RNA outlet to remove one or more RNA molecules;
wherein the first module and the second module together define a flow path along which a sample is capable of flowing.

8. The use of claim 7 wherein:
(i) the oligonucleotides of the first module are linked to a first surface and the oligonucleotides of the second module are linked to a second surface;
(ii) the first module further comprises a sample inlet through which the biological sample is capable of entering the first module;
(iii) the first module further comprises a first reagent inlet through which reagents are capable of entering the first module and/or wherein the second module further comprises a second reagent inlet through which reagents are capable of entering the second module;
(iv) the RNA processing device further comprises temperature control means for adjusting the temperature of the first module and/or the second module;
(v) the first module comprises a flow cell and/or the second module comprises a flow cell; and/or
(vi) the oligonucleotides are optimally spaced.

9. Use of a kit for processing an RNA sample in a method according to any of claims 1 to 6, the kit comprising:
(a) one or more magnetic beads, wherein two or more oligo-dT molecules are linked to the one or more magnetic beads;
(b) a hybridization buffer; and
(c) a reverse transcriptase.

10. The use of claim 9 wherein the kit further comprises one or more, up to all, of dinucleotide triphosphates (dNTPs), MgCl₂ and a buffer.

11. A method of analysing a biological sample from a subject, the method comprising:
(a) extracting RNA from the biological sample;
(b) preparing a processed RNA sample; and
(c) sequencing the processed RNA;
wherein preparing a processed RNA sample comprises taking a portion of the extracted RNA to be a first RNA sample and a portion of the extracted RNA to be a second RNA sample and following the steps of the method of any of claims 1 to 3.

12. The method of claim 11 wherein:
(i) the RNA is full length RNA;
(ii) the biological sample comprises a biological fluid or a fluid or lysate generated from a biological material, optionally wherein the biological fluid comprises a blood sample; and/or
(iii) the RNA extracted from the sample comprises cell-free RNA.

13. The method of any of claims 11 to 12 wherein the method comprises diagnosing a disease in the subject,
optionally wherein the presence or absence of one or more RNA molecules in the processed RNA sample is used to identify whether the subject has the disease.

14. The method of any of claims 11 to 13 wherein extracting RNA from the biological sample comprises:
(a) contacting the biological sample with an oligonucleotide array wherein the oligonucleotide array comprises two or more oligonucleotides linked to a surface and wherein one or more RNA molecules from the sample anneals to the oligonucleotides of the oligonucleotide array;
(b) removing the unannealed sample from the surface; and
(c) disassociating the annealed RNA molecules from the oligonucleotides thereby generating an RNA sample,
optionally wherein the oligonucleotides comprise one or more oligo-dT sequences.

15. The method of any of claims 11 to 14 comprising use of the RNA processing device of any of claims 7 to 8 or the kit according to claim 9 or 10.

## Patentansprüche

1. Verfahren zum Prozessieren von RNA, umfassend:
(i) Kontaktieren einer ersten RNA-Probe mit einem Oligonukleotid-Array, wobei das Oligonukleotid-Array zwei oder mehr an eine Oberfläche gebundene Oligonukleotide umfasst und wobei zwei oder mehr RNA-Moleküle aus der ersten RNA-Probe an die Oligonukleotide des Oligonukleotid-Arrays hybridisieren;
(ii) Verlängern von zwei oder mehr von den Oligonukleotiden durch reverse Transkription unter Verwendung der hybridisierten RNA-Moleküle als Matrizen, um ein DNA-Array zu erzeugen, das zwei oder mehr cDNA-Moleküle umfasst;
(iii) Trennen der hybridisierten RNA-Moleküle von den cDNA-Molekülen;
(iv) Entfernen der ersten RNA-Probe von der Oberfläche;
(v) Kontaktieren einer zweiten RNA-Probe mit dem DNA-Array, wobei ein oder mehrere RNA-Moleküle aus der zweiten RNA-Probe an die cDNA-Moleküle hybridisieren; und
(vi) Extrahieren der nicht hybridisierten RNA-Moleküle, dadurch Erzeugen von prozessierter RNA;
wobei die erste RNA-Probe und die zweite RNA-Probe aus derselben RNA-Probe abgeleitet werden.

2. Verfahren nach Anspruch 1, wobei:
(i) die Oligonukleotide Oligo-dT-Sequenzen umfassen;
(ii) die Oberfläche ein oder mehrere Magnetkügelchen ist;
(iii) das Verfahren in einer mikrofluidischen Durchflusszelle durchgeführt wird;
(iv) die erste RNA-Probe und/oder die zweite RNA-Probe RNA in voller Länge umfassen und/oder
(v) die Oligonukleotide optimal beabstandet sind, sodass die cDNA-Moleküle des DNA-Arrays nicht miteinander interagieren.

3. Verfahren nach Anspruch 1 oder Anspruch 2, weiter umfassend das Sequenzieren der prozessierten RNA.

4. Verfahren nach einem der Ansprüche 1 bis 3, weiter umfassend, vor Schritt (i):
(a) Kontaktieren einer biologischen Probe mit einem Oligonukleotid-Array, wobei das Oligonukleotid-Array zwei oder mehr an eine Oberfläche gebundene Oligonukleotide umfasst und wobei ein oder mehrere RNA-Moleküle aus der biologischen Probe an die Oligonukleotide des Oligonukleotid-Arrays hybridisieren;
(b) Entfernen der nicht hybridisierten Probe von der Oberfläche und
(c) Trennen des/der hybridisierten RNA-Moleküle(s) von den Oligonukleotiden, um eine RNA-Probe zu erhalten.

5. Verfahren nach einem der Ansprüche 1 bis 4, weiter umfassend, anschließend an Schritt (vi), das Trennen der hybridisierten RNA-Moleküle von den cDNA-Molekülen und das Entfernen der getrennten RNA-Moleküle von der Oberfläche und, wahlweise, das Wiederholen der Schritte (v) und (vi) mit einer weiteren RNA-Probe.

6. Verfahren nach Anspruch 4 oder Anspruch 5, wobei die biologische Probe eine biologische Flüssigkeit oder eine Flüssigkeit oder ein Lysat, erzeugt aus einem biologischem Material, umfasst.

7. Verwendung einer RNA-Prozessierungsvorrichtung zum Herstellen von prozessierter RNA aus einer biologischen Probe in einem Verfahren nach einem der Ansprüche 1 bis 6, wobei die Vorrichtung Folgendes umfasst:
(i) ein erstes Modul zum Aufnehmen der biologischen Probe, wobei das erste Modul Folgendes umfasst:
(a) zwei oder mehr Oligonukleotide, die in der Lage sind, an ein oder mehrere RNA-Moleküle in der biologischen Probe zu hybridisieren;
(b) einen ersten Abfallauslass zum Entfernen der nicht hybridisierten Probe;
(c) einen Probenauslass, durch den ein Teil der Probe, der das eine oder die mehreren RNA-Moleküle umfasst, anschließend an die Trennung von den Oligonukleotiden strömen kann; und
(ii) ein zweites Modul zum Aufnehmen des einen oder der mehreren RNA-Moleküle aus dem ersten Modul, wobei das zweite Modul Folgendes umfasst:
(a) zwei oder mehr Oligonukleotide, die in der Lage sind, an ein oder mehrere RNA-Moleküle in der Probe zu hybridisieren;
(b) einen Auslass für prozessierte RNA, durch den die prozessierte RNA erhalten werden kann;
(c) einen Abfall-RNA-Auslass, um ein oder mehrere RNA-Moleküle zu entfernen;
wobei das erste Modul und das zweite Modul zusammen eine Strömungsbahn definieren, entlang derer eine Probe strömen kann.

8. Verwendung nach Anspruch 7, wobei:
(i) die Oligonukleotide des ersten Moduls an eine erste Oberfläche gebunden sind und die Oligonukleotide des zweiten Moduls an eine zweite Oberfläche gebunden sind;
(ii) das erste Modul weiter einen Probeneinlass umfasst, durch den die biologische Probe in das erste Modul eintreten kann;
(iii) das erste Modul weiter einen ersten Reagenseinlass umfasst, durch den Reagenzien in das erste Modul eintreten können, und/oder wobei das zweite Modul weiter einen zweiten Reagenseinlass umfasst, durch den Reagenzien in das zweite Modul eintreten können;
(iv) die RNA-Prozessierungsvorrichtung weiter ein Temperaturregelungsmittel zum Anpassen der Temperatur des ersten Moduls und/oder des zweiten Moduls umfasst;
(v) das erste Modul eine Durchflusszelle umfasst und/oder das zweite Modul eine Durchflusszelle umfasst und/oder
(vi) die Oligonukleotide optimal beabstandet sind.

9. Verwendung eines Kits zum Prozessieren einer RNA-Probe in einem Verfahren nach einem der Ansprüche 1 bis 6, wobei das Kit Folgendes umfasst:
(a) ein oder mehrere magnetische Kügelchen, wobei zwei oder mehr Oligo-dT-Moleküle an das eine oder die mehreren magnetischen Kügelchen gebunden sind;
(b) einen Hybridisierungspuffer und
(c) eine Reverse Transkriptase.

10. Verwendung nach Anspruch 9, wobei das Kit weiter eines oder mehrere, bis zu allen, von Dinukleotidtriphosphaten (dNTPs), MgCl₂ und einem Puffer umfasst.

11. Verfahren zum Analysieren einer biologischen Probe von einem Subjekt, wobei das Verfahren Folgendes umfasst:
(a) Extrahieren von RNA aus der biologischen Probe;
(b) Bereiten einer prozessierten RNA-Probe und
(c) Sequenzieren der prozessierten RNA;
wobei das Bereiten einer prozessierten RNA-Probe das Nehmen eines Teils der extrahierten RNA als eine erste RNA-Probe und eines Teils der extrahierten RNA als eine zweite RNA-Probe und das Befolgen der Schritte des Verfahrens nach einem der Ansprüche 1 bis 3 umfasst.

12. Verfahren nach Anspruch 11, wobei:
(i) die RNA RNA in voller Länge ist;
(ii) die biologische Probe eine biologische Flüssigkeit oder eine Flüssigkeit oder ein Lysat, gewonnen aus biologischem Material, umfasst, wobei die biologische Flüssigkeit wahlweise eine Blutprobe umfasst; und/oder
(iii) die aus der Probe extrahierte RNA zellfreie RNA umfasst.

13. Verfahren nach einem der Ansprüche 11 bis 12, wobei das Verfahren das Diagnostizieren einer Krankheit bei dem Subjekt umfasst,
wobei das Vorhandensein oder Fehlen eines oder mehrerer RNA-Moleküle in der prozessierten RNA-Probe wahlweise dazu verwendet wird, festzustellen, ob das Subjekt an der Krankheit leidet.

14. Verfahren nach einem der Ansprüche 11 bis 13, wobei das Extrahieren von RNA aus der biologischen Probe Folgendes umfasst:
(a) Kontaktieren der biologischen Probe mit einem Oligonukleotid-Array, wobei das Oligonukleotid-Array zwei oder mehr an eine Oberfläche gebundene Oligonukleotide umfasst und wobei ein oder mehrere RNA-Moleküle aus der Probe an die Oligonukleotide des Oligonukleotid-Arrays hybridisieren;
(b) Entfernen der nicht hybridisierten Probe von der Oberfläche und
(c) Trennen der hybridisierten RNA-Moleküle von den Oligonukleotiden, dadurch Erzeugen einer RNA-Probe,
wobei die Oligonukleotide eine oder mehrere Oligo-dT-Sequenzen wahlweise umfassen.

15. Verfahren nach einem der Ansprüche 11 bis 14, umfassend die Verwendung der RNA-Prozessierungsvorrichtung nach einem der Ansprüche 7 bis 8 oder des Kits nach Anspruch 9 oder 10.

## Revendications

1. Procédé de traitement d'ARN comprenant :
(i) la mise en contact d'un premier échantillon d'ARN avec un réseau d'oligonucléotides, dans lequel le réseau d'oligonucléotides comprend deux ou plusieurs oligonucléotides liés à une surface et dans lequel deux ou plusieurs molécules d'ARN du premier échantillon d'ARN s'hybrident aux oligonucléotides du réseau d'oligonucléotides ;
(ii) l'allongement de deux ou plusieurs oligonucléotides par transcription inverse en utilisant les molécules d'ARN hybridées comme matrices pour générer une puce à ADN comprenant deux ou plusieurs molécules d'ADNc ;
(iii) la séparation entre les molécules d'ARN hybridées et les molécules d'ADNc ;
(iv) l'élimination du premier échantillon d'ARN de la surface ;
(v) la mise en contact d'un second échantillon d'ARN avec la puce à ADN, dans lequel une ou plusieurs molécules d'ARN du second échantillon d'ARN s'hybrident aux molécules d'ADNc ; et
(vi) l'extraction des molécules d'ARN non hybridées générant ainsi de l'ARN traité ;
dans lequel le premier échantillon d'ARN et le second échantillon d'ARN sont issus du même échantillon d'ARN.

2. Procédé selon la revendication 1, dans lequel :
(i) les oligonucléotides comprennent des séquences oligo-dT ;
(ii) la surface est constituée d'une ou plusieurs billes magnétiques ;
(iii) le procédé est mis en œuvre dans une cuve à circulation microfluidique ;
(iv) le premier échantillon d'ARN et/ou le second échantillon d'ARN comprennent de l'ARN pleine longueur ; et/ou
(v) les oligonucléotides sont espacés de manière optimale afin que les molécules d'ADNc de la puce à ADN n'interagissent pas entre elles.

3. Procédé selon la revendication 1 ou la revendication 2 comprenant en outre le séquençage de l'ARN traité.

4. Procédé selon l'une quelconque des revendications 1 à 3, comprenant en outre, préalablement à l'étape (i):
(a) la mise en contact d'un échantillon biologique avec un réseau d'oligonucléotides, dans lequel le réseau d'oligonucléotides comprend deux ou plusieurs oligonucléotides liés à une surface et dans lequel une ou plusieurs molécules d'ARN de l'échantillon biologique s'hybrident aux oligonucléotides du réseau d'oligonucléotides ;
(b) l'élimination de l'échantillon non hybridé de la surface ; et
(c) la séparation entre la ou les molécules d'ARN hybridées et les oligonucléotides pour obtenir un échantillon d'ARN.

5. Procédé selon l'une quelconque des revendications 1 à 4 comprenant en outre, après l'étape (vi), la séparation entre les molécules d'ARN hybridées et les molécules d'ADNc et l'élimination des molécules d'ARN séparées de la surface et, éventuellement, la répétition des étapes (v) et (vi) avec un autre échantillon d'ARN.

6. Procédé selon la revendication 4 ou la revendication 5, dans lequel l'échantillon biologique comprend un fluide biologique ou un fluide ou lysat généré à partir d'un matériau biologique.

7. Utilisation d'un dispositif de traitement de l'ARN pour produire de l'ARN traité à partir d'un échantillon biologique selon un procédé selon l'une quelconque des revendications 1 à 6, le dispositif comprenant :
(i) un premier module destiné à recevoir l'échantillon biologique, dans laquelle le premier module comprend :
(a) deux ou plusieursoligonucléotides aptes à s'hybrider à une ou plusieurs molécules d'ARN dans l'échantillon biologique ;
(b) un premier trou d'évacuation pour l'évacuation de l'échantillon non hybridé ;
(c) un orifice de sortie d'échantillon par lequel une partie de l'échantillon comprenant ladite ou lesdites molécules d'ARN peut s'écouler après séparation d'avec les oligonucléotides ; et
(ii) un second module destiné à recevoir ladite ou lesdites molécules d'ARN provenant du premier module, dans laquelle le second module comprend :
(a) deux ou plusieurs oligonucléotides aptes à s'hybrider à une ou plusieurs molécules d'ARN dans l'échantillon ;
(b) un orifice de sortie d'ARN traité par lequel l'ARN traité peut être obtenu ;
(c) un trou d'évacuation de l'ARN usé pour l'évacuation d'une ou plusieurs molécules d'ARN ;
dans laquelle le premier module et le second module délimitent conjointement un trajet d'écoulement le long duquel un échantillon peut circuler.

8. Utilisation selon la revendication 7 dans laquelle :
(i) les oligonucléotides du premier module sont liés à une première surface et les oligonucléotides du second module sont liés à une seconde surface ;
(ii) le premier module comprend en outre un orifice d'entrée d'échantillon par lequel l'échantillon biologique peut pénétrer dans le premier module ;
(iii) le premier module comprend en outre un premier orifice d'entrée de réactifs par lequel les réactifs peuvent pénétrer dans le premier module et/ou dans laquelle le second module comprend en outre un second orifice d'entrée de réactifs par lequel les réactifs peuvent pénétrer dans le second module ;
(iv) le dispositif de traitement de l'ARN comprend en outre un moyen de régulation de la température pour l'ajustement de la température du premier module et/ou du second module ;
(v) le premier module comprend une cuve à circulation et/ou le second module comprend une cuve à circulation ; et/ou
(vi) les oligonucléotides sont espacés de manière optimale.

9. Utilisation d'un kit pour le traitement d'un échantillon d'ARN selon un procédé selon l'une quelconque des revendications 1 à 6, le kit comprenant :
(a) une ou plusieurs billes magnétiques, dans laquelle deux ou plusieurs molécules oligo-dT sont liées à la ou aux billes magnétiques ;
(b) un tampon d'hybridation ; et
(c) une transcriptase inverse.

10. Utilisation selon la revendication 9, dans laquelle le kit comprend en outre un ou plusieurs, et jusqu'à la totalité, des éléments suivants : des dinucléotides triphosphates (dNTP), du MgCl₂ et un tampon.

11. Procédé d'analyse d'un échantillon biologique provenant d'un sujet, le procédé comprenant :
(a) l'extraction de l'ARN hors de l'échantillon biologique ;
b) la préparation d'un échantillon d'ARN traité ; et
(c) le séquençage de l'ARN traité ;
dans lequel la préparation d'un échantillon d'ARN traité comprend le prélèvement d'une partie de l'ARN extrait pour constituer un premier échantillon d'ARN et d'une autre partie de l'ARN extrait pour constituer un second échantillon d'ARN, et la mise en œuvre des étapes du procédé selon l'une quelconque des revendications 1 à 3.

12. Procédé selon la revendication 11, dans lequel :
(i) l'ARN est un ARN pleine longueur ;
(ii) l'échantillon biologique comprend un fluide biologique ou un fluide ou lysat généré à partir d'un matériau biologique, dans lequel le fluide biologique comprend éventuellement un échantillon de sang ; et/ou
(iii) l'ARN extrait de l'échantillon comprend de l'ARN acellulaire.

13. Procédé selon l'une quelconque des revendications 11 à 12, dans lequel le procédé comprend le diagnostic d'une maladie chez le sujet,
éventuellement dans lequel la présence ou l'absence d'une ou plusieurs molécules d'ARN dans l'échantillon d'ARN traité est utilisée pour déterminer si le sujet est atteint ou non de la maladie.

14. Procédé selon l'une quelconque des revendications 11 à 13 dans lequel l'extraction de l'ARN hors de l'échantillon biologique comprend :
(a) la mise en contact de l'échantillon biologique avec un réseau d'oligonucléotides dans lequel le réseau d'oligonucléotides comprend deux ou plusieurs oligonucléotides liés à une surface et dans lequel une ou plusieurs molécules d'ARN de l'échantillon s'hybrident aux oligonucléotides du réseau d'oligonucléotides ;
b) l'élimination de l'échantillon non hybridé de la surface ; et
(c) la séparation entre les molécules d'ARN hybridées et les oligonucléotides, générant ainsi un échantillon d'ARN,
éventuellement dans lequel les oligonucléotides comprennent une ou plusieurs séquences oligo-dT.

15. Procédé selon l'une quelconque des revendications 11 à 14 comprenant l'utilisation du dispositif de traitement de l'ARN selon l'une quelconque des revendications 7 à 8 ou du kit selon la revendication 9 ou 10.
